# EUROPEAN PATENT SPECIFICATION

(11) **EP 1 895 001 B1**
(45) Date of publication and mention of the grant of the patent: **03.08.2011**
(21) Application number: 07023492.7
(22) Date of filing: 02.11.1999
(51) Int. Cl.: C12N 9/64, C12N 15/57, C12N 5/10, C12P 21/02, G01N 33/573, C07K 16/40, C12P 21/08, C12Q 1/37, C12R 1/91

(54) **Novel trypsin family serine proteases**
Neue Serinproteasen der Trypsin-Familie
Nouvelles sérines protéases de la famille de la trypsine

(30) Priority: 04.11.1998 JP 31336698
(43) Date of publication of application: 05.03.2008
(62) Divisional of application: 99951213.0
(73) Proprietor: CHUGAI SEIYAKU KABUSHIKI KAISHA, Tokyo, 115-8543 (JP)
(72) Inventor: Senoo, Chiaki, Kanagawa 247-8530 (JP); Numata, Mariko, Kanagawa 247-8530 (JP)
(74) Representative: Vossius & Partner

(56) References cited:
- WO-A-98/36054
- CHUNG,D.W., FUJIKAWA,K., MCMULLEN,B.A., DAVIE,E.W.,: "Human plasma prekallikrein, a zymogen to a serine protease that contains four tandem repeats." BIOCHEMISTRY, vol. 25, no. 9, 1986, pages 2410-2407, XP001056849
- MCMULLEN,B.A., FUJIKAWA,K., DAVIE,E.W.: "Location of the disulfide bonds in human plasma prekallikrein: the presence of four novel apple domains in the amino-terminal portion of the molecule." BIOCHEMISTRY, vol. 30, no. 8, 1991, pages 2050-2056, XP002190836
- LUM LAWRENCE ET AL: "Evidence for distinct serine protease activities with a potential role in processing the sperm protein fertilin" DEVELOPMENTAL BIOLOGY, vol. 191, no. 1, 1 November 1997 (1997-11-01), pages 131-145, XP009095029 ISSN: 0012-1606

### SEQUENCE LISTING

<110> CHUGAI SEIYAKU KABUSHIFCI KAISHA
<120> NOVEL TRYPSIN FAMILY SERINE PROTEASES
<130> F1790 EP/2 S3
<150> EP 99 95 1213.0 <151> 1999-11-02
<150> PCT/JP99/06111 <151> 1999-11-02
<150> JP 1998-313366 <151> 1998-11-04
<160> 45
<210> 1
   <211> 1033
   <212> DNA
   <213> Mus musculus
<220>
   <221> CDS
   <222> (48)..(1010)
<400> 1
   cctgcctcag tgttggagct ccccattgct gatgtgcagg caagccg atg aaa cga      56
                                                       Met Lys Arg
                                                       1
   tgg aag gac aga aga aca ggc ctg ttg ctg cca ttg gtc ctc ctg ttg     104
   Trp Lys Asp Arg Arg Thr Gly Leu Leu Leu Pro Leu Val Leu Leu Leu
       5                   10                  15
   ttt ggg gca tgt agc tca ctg gca tgg gta tgt ggc cgg cga atg agt     152
   Phe Gly Ala Cys Ser Ser Leu Ala Trp Val Cys Gly Arg Arg Met Ser
   20                  25                  30                  35
   agc aga tcc caa caa ctt aac aat gct tct gct atc gtg gaa ggc aaa     200
   Ser Arg Ser Gln Gln Leu Asn Asn Ala Ser Ala Ile Val Glu Gly Lys
                   40                  45                  50
   cct gct tct gct atc gtg gga ggc aaa cct gca aac atc ttg gag ttc     248
   Pro Ala Ser Ala Ile Val Gly Gly Lys Pro Ala Asn Ile Leu Glu Phe
               55                  60                  65
   ccc tgg cat gtg ggg att atg aat cat ggt agt cat ctc tgt ggg gga     296
   Pro Trp His Val Gly Ile Met Asn His Gly Ser His Leu Cys Gly Gly
           70                  75                  80
   tct att ctc aat gag tgg tgg gtt cta tct gca tcc cat tgc ttc gac     344
   Ser Ile Leu Asn Glu Trp Trp Val Leu Ser Ala Ser His Cys Phe Asp
       85                  90                  95
   caa cta aac aac tct aaa ttg gag atc att cat ggc act gaa gac ctc     392
   Gln Leu Asn Asn Ser Lys Leu Glu Ile Ile His Gly Thr Glu Asp Leu
   100                 105                 110                 115
   agc aca aag ggc ata aag tat cag aaa gtg gac aag tta ttc ttg cac      440
   Ser Thr Lys Gly Ile Lys Tyr Gln Lys Val Asp Lys Leu Phe Leu His
                   120                 125                 130
   cca aag ttt gat gac tgg ctc ctg gac aac gac ata gct ttg ctc ttg      488
   Pro Lys Phe Asp Asp Trp Leu Leu Asp Asn Asp Ile Ala Leu Leu Leu
               135                 140                 145
   ctc aaa tcc cca tta aac ttg agt gtc aac agg ata cct atc tgc act      536
   Leu Lys Ser Pro Leu Asn Leu Ser Val Asn Arg Ile Pro Ile Cys Thr
           150                 155                 160
   tca gaa atc tct gac ata cag gca tgg agg aac tgc tgg gtg aca gga      584
   Ser Glu Ile Ser Asp Ile Gln Ala Trp Arg Asn Cys Trp Val Thr Gly
       165                 170                 175
   tgg ggc att act aat act agt gaa aaa gga gtc caa ccc aca att ctg      632
   Trp Gly Ile Thr Asn Thr Ser Glu Lys Gly Val Gln Pro Thr Ile Leu
   180                 185                 190                 195
   cag gca gtc aaa gtg gat ctg tac aga tgg gat tgg tgt ggc tat att      680
   Gln Ala Val Lys Val Asp Leu Tyr Arg Trp Asp Trp Cys Gly Tyr Ile
                   200                 205                 210
   ttg tct cta tta acc aag aat atg ctg tgt gct ggg act caa gat cct      728
   Leu Ser Leu Leu Thr Lys Asn Met Leu Cys Ala Gly Thr Gln Asp Pro
               215                 220                 225
   ggg aag gat gcc tgc cag ggc gac agt gga gga gct ctc gtt tgc aac      776
   Gly Lys Asp Ala Cys Gln Gly Asp Ser Gly Gly Ala Leu Val Cys Asn
           230                 235                 240
   aaa aag aga aac aca gcc att tgg tac cag gtg ggc att gtc agc tgg      824
   Lys Lys Arg Asn Thr Ala Ile Trp Tyr Gln Val Gly Ile Val Ser Trp
       245                 250                 255
   ggc atg ggc tgt ggc aag aag aat ctg cca gga gta tac acc aag gtg      872
   Gly Met Gly Cys Gly Lys Lys Asn Leu Pro Gly Val Tyr Thr Lys Val
   260                 265                 270                 275
   tca cac tat gtg agg tgg atc agc aag cag aca gcg aag gcg ggg agg      920
   Ser His Tyr Val Arg Trp Ile Ser Lys Gln Thr Ala Lys Ala Gly Arg
                   280                 285                 290
   cct tat atg tat gag cag aac tct gcg tgc cct ttg gtg ctc tct tgc      968
   Pro Tyr Met Tyr Glu Gln Asn Ser Ala Cys Pro Leu Val Leu Ser Cys
               295                 300                 305
   cgg gct atc ttg ttc cta tat ttt gta atg ttt ctt cta acc tga         1013
   Arg Ala Ile Leu Phe Leu Tyr Phe Val Met Phe Leu Leu Thr
           310                 315                 320
   tgattaaacg tgagactgcc                                               1033
<210> 2
   <211> 321
   <212> PRT
   <213> Mus musculus
<400> 2
   Met Lys Arg
    1
   Trp Lys Asp Arg Arg Thr Gly Leu Leu Leu Pro Leu Val Leu Leu Leu
        5                  10                  15
   Phe Gly Ala Cys Ser Ser Leu Ala Trp Val Cys Gly Arg Arg Met Ser
    20                 25                  30                  35
   Ser Arg Ser Gln Gln Leu Asn Asn Ala Ser Ala Ile Val Glu Gly Lys
                   40                  45                  50
   Pro Ala Ser Ala Ile Val Gly Gly Lys Pro Ala Asn Ile Leu Glu Phe
               55                  60                  65
   Pro Trp His Val Gly Ile Met Asn His Gly Ser His Leu Cys Gly Gly
           70                  75                  80
   Ser Ile Leu Asn Glu Trp Trp Val Leu Ser Ala Ser His Cys Phe Asp
       85                  90                  95
   Gln Leu Asn Asn Ser Lys Leu Glu Ile Ile His Gly Thr Glu Asp Leu
   100                 105                 110                 115
   Ser Thr Lys Gly Ile Lys Tyr Gln Lys Val Asp Lys Leu Phe Leu His
                   120                 125                 130
   Pro Lys Phe Asp Asp Trp Leu Leu Asp Asn Asp Ile Ala Leu Leu Leu
               135                 140                 145
   Leu Lys Ser Pro Leu Asn Leu Ser Val Asn Arg Ile Pro Ile Cys Thr
           150                 155                 160
   Ser Glu Ile Ser Asp Ile Gln Ala Trp Arg Asn Cys Trp Val Thr Gly
       165                 170                 175
   Trp Gly Ile Thr Asn Thr Ser Glu Lys Gly Val Gln Pro Thr Ile Leu
   180                 185                 190                 195
   Gln Ala Val Lys Val Asp Leu Tyr Arg Trp Asp Trp Cys Gly Tyr Ile
                   200                 205                 210
   Leu Ser Leu Leu Thr Lys Asn Met Leu Cys Ala Gly Thr Gln Asp Pro
               215                 220                 225
   Gly Lys Asp Ala Cys Gln Gly Asp Ser Gly Gly Ala Leu Val Cys Asn
           230                 235                 240
   Lys Lys Arg Asn Thr Ala Ile Trp Tyr Gln Val Gly Ile Val Ser Trp
       245                 250                 255
   Gly Met Gly Cys Gly Lys Lys Asn Leu Pro Gly Val Tyr Thr Lys Val
   260                 265                 270                 275
   Ser His Tyr Val Arg Trp Ile Ser Lys Gln Thr Ala Lys Ala Gly Arg
                   280                 285                 290
   Pro Tyr Met Tyr Glu Gln Asn Ser Ala Cys Pro Leu Val Leu Ser Cys
               295                 300                 305
   Arg Ala Ile Leu Phe Leu Tyr Phe Val Met Phe Leu Leu Thr
           310                 315                 320
<210> 3
   <211> 1034
   <212> DNA
   <213> Mus musculus
<220>
   <221> CDS
   <222> (69)..(1025)
<400> 3
   cccacgcgtn cggttgtatc aatgtgggca gggcatcaag gcaggcacca ctgcactgga   60
   atgacaac atg atg ctc cca ctt cta att gca ctg ctc atg gct tcc aag   110
            Met Met Leu Pro Leu Leu Ile Ala Leu Leu Met Ala Ser Lys
            1               5                   10
   gga caa gct aag gac cag caa gaa tca gtt ctg tgt ggc cac aga cct    158
   Gly Gln Ala Lys Asp Gln Gln Glu Ser Val Leu Cys Gly His Arg Pro
   15                  20                  25                  30
   gcc ttc cca aac tca tca tgg ctg cca ttg cgg gag ctg ctt gag gtc    206
   Ala Phe Pro Asn Ser Ser Trp Leu Pro Leu Arg Glu Leu Leu Glu Val
                   35                  40                  45
   cag cat ggt gag ttc cca tgg caa gtg agt atc cag atg ctt ggg aaa    254
   Gln His Gly Glu Phe Pro Trp Gln Val Ser Ile Gln Met Leu Gly Lys
               50                  55                  60
   cac ctg tgt gga ggc tcc atc atc cac cgg tgg tgg gtt ctg aca gca    302
   His Leu Cys Gly Gly Ser Ile Ile His Arg Trp Trp Val Leu Thr Ala
           65                  70                  75
   gca cac tgc ttc ccg aga acc cta tta gaa ctg gta gca gtc aat gtc    350
   Ala His Cys Phe Pro Arg Thr Leu Leu Glu Leu Val Ala Val Asn Val
       80                  85                  90
   act gtg gtc atg gga atc aag act ttc agt gac acc aac tta gag aga    398
   Thr Val Val Met Gly Ile Lys Thr Phe Ser Asp Thr Asn Leu Glu Arg
   95                  100                 105                 110
   aaa caa gtg cag aag atc att gct cac aga gac tac aaa ccg ccc gac    446
   Lys Gln Val Gln Lys Ile Ile Ala His Arg Asp Tyr Lys Pro Pro Asp
                   115                 120                 125
   ctt gac agc gac ctc tgc ctg ctc cta ctt gcc acg cca atc caa ttc    494
   Leu Asp Ser Asp Leu Cys Leu Leu Leu Leu Ala Thr Pro Ile Gln Phe
               130                 135                 140
   aat aaa gac aaa atg ccc atc tgc ctg cca cag agg gag aac tcc tgg    542
   Asn Lys Asp Lys Met Pro Ile Cys Leu Pro Gln Arg Glu Asn Ser Trp
           145                 150                 155
   gac cgg tgc tgg atg tca gag tgg gca tat act cat ggc cat ggt tca    590
   Asp Arg Cys Trp Met Ser Glu Trp Ala Tyr Thr His Gly His Gly Ser
       160                 165                 170
   gcc aaa ggc tca aac atg cac ctg aag aag ctc agg gtg gtt cag att      638
   Ala Lys Gly Ser Asn Met His Leu Lys Lys Leu Arg Val Val Gln Ile
   175                 180                 185                 190
   agc tgg agg aca tgt gcg aag agg gtg act cag ctc tcc agg aac atg      686
   Ser Trp Arg Thr Cys Ala Lys Arg Val Thr Gln Leu Ser Arg Asn Met
                   195                 200                 205
   ctt tgt gct tgg aag gaa gtg ggc acc aac ggc aag tgc cag gga gac      734
   Leu Cys Ala Trp Lys Glu Val Gly Thr Asn Gly Lys Cys Gln Gly Asp
               210                 215                 220
   agc ggg gca ccc atg gtc tgt gct aac tgg gag act cgg aga ctc ttt      782
   Ser Gly Ala Pro Met Val Cys Ala Asn Trp Glu Thr Arg Arg Leu Phe
           225                 230                 235
   caa gtg ggt gtc ttc agc tgg ggc ata act tca gga tcc agg ggg agg      830
   Gln Val Gly Val Phe Ser Trp Gly Ile Thr Ser Gly Ser Arg Gly Arg
       240                 245                 250
   cca ggc att ttt gtg tct gtg gct cag ttt atc cca tgg atc ctg gag      878
   Pro Gly Ile Phe Val Ser Val Ala Gln Phe Ile Pro Trp Ile Leu Glu
   255                 260                 265                 270
   gag aca caa agg gag gga cga gcc ctt gcc ctc tca aag gcc tca aaa      926
   Glu Thr Gln Arg Glu Gly Arg Ala Leu Ala Leu Ser Lys Ala Ser Lys
                   275                 280                 285
   agt ctc ttg gct ggc agt cca cgc tac cat ccc ata ttg cta agc atg      974
   Ser Leu Leu Ala Gly Ser Pro Arg Tyr His Pro Ile Leu Leu Ser Met
               290                 295                 300
   ggc tct caa ata ctg ctt gct gcc ata ttt tct gat gat aaa tca aat     1022
   Gly Ser Gln Ile Leu Leu Ala Ala Ile Phe Ser Asp Asp Lys Ser Asn
           305                 310                 315
   tgc taa gctctg                                                      1034
   Cys
<210> 4
   <211> 319
   <212> PRT
   <213> Mus musculus
<400> 4
   Met Met Leu Pro Leu Leu Ile Ala Leu Leu Met Ala Ser Lys
   1               5                   10
   Gly Gln Ala Lys Asp Gln Gln Glu Ser Val Leu Cys Gly His Arg Pro
   15                  20                  25                  30
   Ala Phe Pro Asn Ser Ser Trp Leu Pro Leu Arg Glu Leu Leu Glu Val
                   35                  40                  45
   Gln His Gly Glu Phe Pro Trp Gln Val Ser Ile Gln Met Leu Gly Lys
               50                  55                  60
   His Leu Cys Gly Gly Ser Ile Ile His Arg Trp Trp Val Leu Thr Ala
           65                  70                  75
   Ala His Cys Phe Pro Arg Thr Leu Leu Glu Leu Val Ala Val Asn Val
       80                  85                  90
   Thr Val Val Met Gly Ile Lys Thr Phe Ser Asp Thr Asn Leu Glu Arg
    95                 100                 105                 110
   Lys Gln Val Gln Lys Ile Ile Ala His Arg Asp Tyr Lys Pro Pro Asp
                   115                 120                 125
   Leu Asp Ser Asp Leu Cys Leu Leu Leu Leu Ala Thr Pro Ile Gln Phe
               130                 135                 140
   Asn Lys Asp Lys Met Pro Ile Cys Leu Pro Gln Arg Glu Asn Ser Trp
           145                 150                 155
   Asp Arg Cys Trp Met Ser Glu Trp Ala Tyr Thr His Gly His Gly Ser
       160                 165                 170
   Ala Lys Gly Ser Asn Met His Leu Lys Lys Leu Arg Val Val Gln Ile
   175                 180                 185                 190
   Ser Trp Arg Thr Cys Ala Lys Arg Val Thr Gln Leu Ser Arg Asn Met
                   195                 200                 205
   Leu Cys Ala Trp Lys Glu Val Gly Thr Asn Gly Lys Cys Gln Gly Asp
               210                 215                 220
   Ser Gly Ala Pro Met Val Cys Ala Asn Trp Glu Thr Arg Arg Leu Phe
           225                 230                 235
   Gln Val Gly Val Phe Ser Trp Gly Ile Thr Ser Gly Ser Arg Gly Arg
       240                 245                 250
   Pro Gly Ile Phe Val Ser Val Ala Gln Phe Ile Pro Trp Ile Leu Glu
   255                 260                 265                 270
   Glu Thr Gln Arg Glu Gly Arg Ala Leu Ala Leu Ser Lys Ala Ser Lys
                   275                 280                 285
   Ser Leu Leu Ala Gly Ser Pro Arg Tyr His Pro Ile Leu Leu Ser Met
               290                 295                 300
   Gly Ser Gln Ile Leu Leu Ala Ala Ile Phe Ser Asp Asp Lys Ser Asn
           305                 310                 315
   Cys
<210> 5
   <211> 1035
   <212> DNA
   <213> Homo sapiens
<220>
   <221> CDS
   <222> (73)..(867)
<400> 5
   ctgtggctgg catgttgtca gctctggctg gaggcaaagg tttggcaatt ttggactgga 60
   attgacaaga ag atg ttc cag ctt cta att ccc ctg ctt ttg gca ctc aag  111
                 Met Phe Gln Leu Leu Ile Pro Leu Leu Leu Ala Leu Lys
                  1               5                   10
   gga cat gcc cag gac aat cca gaa aac gta caa tgt ggc cac agg cct     159
   Gly His Ala Gln Asp Asn Pro Glu Asn Val Gln Cys Gly His Arg Pro
        15                  20                  25
   gct ttt cca aac tcg tca tgg tta cca ttt cat gaa cgg ctt caa gtc     207
   Ala Phe Pro Asn Ser Ser Trp Leu Pro Phe His Glu Arg Leu Gln Val
    30                  35                  40                  45
   cag aat ggt gag tgc ccg tgg caa gtg agt atc cag atg tca cgg aaa     255
   Gln Asn Gly Glu Cys Pro Trp Gln Val Ser Ile Gln Met Ser Arg Lys
                    50                  55                  60
   cac ctc tgt gga ggc tca atc tta cat tgg tgg tgg gtt ctg aca gcc     303
   His Leu Cys Gly Gly Ser Ile Leu His Trp Trp Trp Val Leu Thr Ala
                65                  70                  75
   gca cac tgc ttc cga aga acc cta tta gac atg gcc gtg gta aat gtc     351
   Ala His Cys Phe Arg Arg Thr Leu Leu Asp Met Ala Val Val Asn Val
            80                  85                  90
   act gtg gtc atg gga acg aga aca ttc agc aac atc cac tcg gag aga     399
   Thr Val Val Met Gly Thr Arg Thr Phe Ser Asn Ile His Ser Glu Arg
        95                 100                 105
   aag caa gtg cag aag gtc att att cac aaa gat tac aaa ccg ccc cag     447
   Lys Gln Val Gln Lys Val Ile Ile His Lys Asp Tyr Lys Pro Pro Gln
   110                 115                 120                 125
   ctc gac agt gac ctc tct ctg ctt cta ctt gcc aca cca gtg caa ttc     495
   Leu Asp Ser Asp Leu Ser Leu Leu Leu Leu Ala Thr Pro Val Gln Phe
                   130                 135                 140
   agc aat ttc aaa atg cct gtc tgc ctg cag gag gag gag agg acc tgg     543
   Ser Asn Phe Lys Met Pro Val Cys Leu Gln Glu Glu Glu Arg Thr Trp
               145                 150                 155
   gac tgg tgt tgg atg gca cag tgg gta acg acc aat ggg tat gac caa     591
   Asp Trp Cys Trp Met Ala Gln Trp Val Thr Thr Asn Gly Tyr Asp Gln
           160                 165                 170
   tat gat gac tta aac atg cac ctg gaa aag ctg aga gtg gtg cag att     639
   Tyr Asp Asp Leu Asn Met His Leu Glu Lys Leu Arg Val Val Gln Ile
       175                 180                 185
   agc cgg aaa gaa tgt gcc aag agg gta aac cag ctg tcc agg aac atg     687
   Ser Arg Lys Glu Cys Ala Lys Arg Val Asn Gln Leu Ser Arg Asn Met
   190                 195                 200                 205
   att tgt gct tcg aac gaa cca ggc acc aat ggt atc ttc aag gga gac     735
   Ile Cys Ala Ser Asn Glu Pro Gly Thr Asn Gly Ile Phe Lys Gly Asp
                   210                 215                 220
   agt ggg gca cct ctg gtt tgt gct att tat gga acc cag aga ctc ttc     783
   Ser Gly Ala Pro Leu Val Cys Ala Ile Tyr Gly Thr Gln Arg Leu Phe
               225                 230                 235
   caa gtg ggt gtc ttc agt ggg ggc ata aga tct ggc tcc agg ggg aga     831
   Gln Val Gly Val Phe Ser Gly Gly Ile Arg Ser Gly Ser Arg Gly Arg
           240                 245                 250
   cct ggt atg ttt gtg tct gtg gct caa ttt att cca tga agccaggagg      880
   Pro Gly Met Phe Val Ser Val Ala Gln Phe Ile Pro
       255                 260                 265
   agacagaaaa ggaggggaaa gcctacacca taatctcagg atccacgaga agccgagaag  940
   ctcactggtg tgtgttcctc agtacccctt cttgctagga ttggggtctc aaatgctgct 1000
   ggccaccatg tttaccggtg ataaacctaa cyrcw                             1035
<210> 6
   <211> 265
   <212> PRT
   <213> Homo sapiens
<400> 6
   Met Phe Gln Leu Leu Ile Pro Leu Leu Leu Ala Leu Lys
   1               5                   10
   Gly His Ala Gln Asp Asn Pro Glu Asn Val Gln Cys Gly His Arg Pro
       15                  20                  25
   Ala Phe Pro Asn Ser Ser Trp Leu Pro Phe His Glu Arg Leu Gln Val
   30                  35                  40                  45
   Gln Asn Gly Glu Cys Pro Trp Gln Val Ser Ile Gln Met Ser Arg Lys
                   50                  55                  60
   His Leu Cys Gly Gly Ser Ile Leu His Trp Trp Trp Val Leu Thr Ala
               65                  70                  75
   Ala His Cys Phe Arg Arg Thr Leu Leu Asp Met Ala Val Val Asn Val
           80                  85                  90
   Thr Val Val Met Gly Thr Arg Thr Phe Ser Asn Ile His Ser Glu Arg
       95                  100                 105
   Lys Gln Val Gln Lys Val Ile Ile His Lys Asp Tyr Lys Pro Pro Gln
   110                 115                 120                 125
   Leu Asp Ser Asp Leu Ser Leu Leu Leu Leu Ala Thr Pro Val Gln Phe
                   130                 135                 140
   Ser Asn Phe Lys Met Pro Val Cys Leu Gln Glu Glu Glu Arg Thr Trp
               145                 150                 155
   Asp Trp Cys Trp Met Ala Gln Trp Val Thr Thr Asn Gly Tyr Asp Gln
           160                 165                 170
   Tyr Asp Asp Leu Asn Met His Leu Glu Lys Leu Arg Val Val Gln Ile
       175                 180                 185
   Ser Arg Lys Glu Cys Ala Lys Arg Val Asn Gln Leu Ser Arg Asn Met
   190                 195                 200                 205
   Ile Cys Ala Ser Asn Glu Pro Gly Thr Asn Gly Ile Phe Lys Gly Asp
                   210                 215                 220
   Ser Gly Ala Pro Leu Val Cys Ala Ile Tyr Gly Thr Gln Arg Leu Phe
               225                 230                 235
   Gln Val Gly Val Phe Ser Gly Gly Ile Arg Ser Gly Ser Arg Gly Arg
           240                 245                 250
   Pro Gly Met Phe Val Ser Val Ala Gln Phe Ile Pro
       255                 260                 265
<210> 7
   <211> 1028
   <212> DNA
   <213> Mus musculus
<220>
   <221> CDS
   <222> (38)..(1000)
<400> 7
   gtcagcctgg cctccaacac acagcacagc cagagcc atg atc ctg ccc tcc atc      55
                                            Met Ile Leu Pro Ser Ile
                                            1               5
   ctg cta ctt gtt gcc cac acc ctg gaa gca aat gtt gag tgt ggt gtg      103
   Leu Leu Leu Val Ala His Thr Leu Glu Ala Asn Val Glu Cys Gly Val
               10                  15                  20
   aga ccc ctg tat gat agc aga att caa tac tcc agg atc ata gaa ggg      151
   Arg Pro Leu Tyr Asp Ser Arg Ile Gln Tyr Ser Arg Ile Ile Glu Gly
           25                  30                  35
   cag gag gct gag ctg ggt gag ttt cca tgg cag gtg agc att cag gaa      199
   Gln Glu Ala Glu Leu Gly Glu Phe Pro Trp Gln Val Ser Ile Gln Glu
       40                  45                  50
   agt gac cac cat ttc tgc ggc ggc tcc att ctc agt gag tgg tgg atc      247
   Ser Asp His His Phe Cys Gly Gly Ser Ile Leu Ser Glu Trp Trp Ile
   55                  60                  65                  70
   ctc acc gtg gcc cac tgc ttc tat gct cag gag ctt tcc cca aca gat      295
   Leu Thr Val Ala His Cys Phe Tyr Ala Gln Glu Leu Ser Pro Thr Asp
                   75                  80                  85
   ctc aga gtc aga gtg gga acc aat gac tta act act tca ccc gtg gaa      343
   Leu Arg Val Arg Val Gly Thr Asn Asp Leu Thr Thr Ser Pro Val Glu
               90                  95                  100
   cta gag gtc acc acc ata atc cgg cac aaa ggc ttt aaa cgg ctg aac      391
   Leu Glu Val Thr Thr Ile Ile Arg His Lys Gly Phe Lys Arg Leu Asn
           105                 110                 115
   atg gac aac gac att gcc ttg ttg ctg cta gcc aag ccc ttg gcg ttc      439
   Met Asp Asn Asp Ile Ala Leu Leu Leu Leu Ala Lys Pro Leu Ala Phe
       120                 125                 130
   aat gag ctg acg gtg ccc atc tgc ctt cct ctc tgg ccc gcc cct ccc      487
   Asn Glu Leu Thr Val Pro Ile Cys Leu Pro Leu Trp Pro Ala Pro Pro
   135                 140                 145                 150
   agc tgg cac gaa tgc tgg gtg gca gga tgg ggc gta acc aac tca act      535
   Ser Trp His Glu Cys Trp Val Ala Gly Trp Gly Val Thr Asn Ser Thr
                   155                 160                 165
   gac aag gaa tct atg tca acg gat ctg atg aag gtg ccc atg cgt atc      583
   Asp Lys Glu Ser Met Ser Thr Asp Leu Met Lys Val Pro Met Arg Ile
               170                 175                 180
   ata gag tgg gag gaa tgc tta cag atg ttt ccc agc ctc acc aca aac      631
   Ile Glu Trp Glu Glu Cys Leu Gln Met Phe Pro Ser Leu Thr Thr Asn
           185                 190                 195
   atg ctg tgt gcc tca tat ggt aat gag agc tac gat gct tgc cag ggt      679
   Met Leu Cys Ala Ser Tyr Gly Asn Glu Ser Tyr Asp Ala Cys Gln Gly
       200                 205                 210
   gac agt ggg gga ccg ctt gtc tgc acc aca gat cct ggc agt agg tgg      727
   Asp Ser Gly Gly Pro Leu Val Cys Thr Thr Asp Pro Gly Ser Arg Trp
   215                 220                 225                 230
   tac cag gtg ggc atc atc agc tgg ggc aag agc tgt gga aaa aaa ggc      775
   Tyr Gln Val Gly Ile Ile Ser Trp Gly Lys Ser Cys Gly Lys Lys Gly
                   235                 240                 245
   ttc cca ggg ata tat act gta ttg gca aag tat acc ctg tgg att gag      823
   Phe Pro Gly Ile Tyr Thr Val Leu Ala Lys Tyr Thr Leu Trp Ile Glu
               250                 255                 260
   aaa ata gcc cag aca gag ggg aag ccc ctg gat ttt aga ggt cag agc      871
   Lys Ile Ala Gln Thr Glu Gly Lys Pro Leu Asp Phe Arg Gly Gln Ser
           265                 270                 275
   tcc tct aac aag aag aaa aac aga cag aac aat cag ctc tcc aaa tcc      919
   Ser Ser Asn Lys Lys Lys Asn Arg Gln Asn Asn Gln Leu Ser Lys Ser
       280                 285                 290
   cca gcc ctg aac tgc ccc caa agc tgg ctc ctg ccc tgt ctg ctg tcc      967
   Pro Ala Leu Asn Cys Pro Gln Ser Trp Leu Leu Pro Cys Leu Leu Ser
   295                 300                 305                 310
   ttt gca ctg ctt aga gcc ttg tcc aac tgg aaa taaaacaatg cagtctctga   1020
   Phe Ala Leu Leu Arg Ala Leu Ser Asn Trp Lys
                   315                 320
   tccaccct                                                            1028
<210> 8
   <211> 321
   <212> PRT
   <213> Mus musculus
<400> 8
   Met Ile Leu Pro Ser Ile
    1               5
   Leu Leu Leu Val Ala His Thr Leu Glu Ala Asn Val Glu Cys Gly Val
               10                  15                  20
   Arg Pro Leu Tyr Asp Ser Arg Ile Gln Tyr Ser Arg Ile Ile Glu Gly
           25                  30                  35
   Gln Glu Ala Glu Leu Gly Glu Phe Pro Trp Gln Val Ser Ile Gln Glu
       40                  45                  50
   Ser Asp His His Phe Cys Gly Gly Ser Ile Leu Ser Glu Trp Trp Ile
   55                  60                  65                  70
   Leu Thr Val Ala His Cys Phe Tyr Ala Gln Glu Leu Ser Pro Thr Asp
                   75                  80                  85
   Leu Arg Val Arg Val Gly Thr Asn Asp Leu Thr Thr Ser Pro Val Glu
               90                  95                  100
   Leu Glu Val Thr Thr Ile Ile Arg His Lys Gly Phe Lys Arg Leu Asn
           105                 110                 115
   Met Asp Asn Asp Ile Ala Leu Leu Leu Leu Ala Lys Pro Leu Ala Phe
       120                 125                 130
   Asn Glu Leu Thr Val Pro Ile Cys Leu Pro Leu Trp Pro Ala Pro Pro
   135                 140                 145                 150
   Ser Trp His Glu Cys Trp Val Ala Gly Trp Gly Val Thr Asn Ser Thr
                   155                 160                 165
   Asp Lys Glu Ser Met Ser Thr Asp Leu Met Lys Val Pro Met Arg Ile
               170                 175                 180
   Ile Glu Trp Glu Glu Cys Leu Gln Met Phe Pro Ser Leu Thr Thr Asn
           185                 190                 195
   Met Leu Cys Ala Ser Tyr Gly Asn Glu Ser Tyr Asp Ala Cys Gln Gly
       200                 205                 210
   Asp Ser Gly Gly Pro Leu Val Cys Thr Thr Asp Pro Gly Ser Arg Trp
   215                 220                 225                 230
   Tyr Gln Val Gly Ile Ile Ser Trp Gly Lys Ser Cys Gly Lys Lys Gly
                   235                 240                 245
   Phe Pro Gly Ile Tyr Thr Val Leu Ala Lys Tyr Thr Leu Trp Ile Glu
               250                 255                 260
   Lys Ile Ala Gln Thr Glu Gly Lys Pro Leu Asp Phe Arg Gly Gln Ser
           265                 270                 275
   Ser Ser Asn Lys Lys Lys Asn Arg Gln Asn Asn Gln Leu Ser Lys Ser
       280                 285                 290
   Pro Ala Leu Asn Cys Pro Gln Ser Trp Leu Leu Pro Cys Leu Leu Ser
   295                 300                 305                 310
   Phe Ala Leu Leu Arg Ala Leu Ser Asn Trp Lys
                   315                 320
<210> 9
   <211> 1123
   <212> DNA
   <213> Homo sapiens
<220>
   <221> CDS
   <222> (41) .. (1096)
<400> 9
   ggcctctgtc acccccgggc ccacagcaca gcccagggcc atg ctc ctg ttc tca      55
                                               Met Leu Leu Phe Ser
                                                1               5
   gtg ttg ctg ctc ctg tcc ctg gtc acg gga act cag ctc ggt cca cgg     103
   Val Leu Leu Leu Leu Ser Leu Val Thr Gly Thr Gln Leu Gly Pro Arg
                       10                  15                  20
   act cct ctc cca gag gct gga gtg gct atc cta ggc agg gct agg gga     151
   Thr Pro Leu Pro Glu Ala Gly Val Ala Ile Leu Gly Arg Ala Arg Gly
                   25                  30                  35
   gcc cac cgc cct cag ccc cgt cat ccc ccc agc cca gtc agt gaa tgt     199
   Ala His Arg Pro Gln Pro Arg His Pro Pro Ser Pro Val Ser Glu Cys
               40                  45                  50
   ggt gac aga tct att ttc gag gga aga act cgg tat tcc aga atc aca     247
   Gly Asp Arg Ser Ile Phe Glu Gly Arg Thr Arg Tyr Ser Arg Ile Thr
           55                  60                  65
   ggg ggg atg gag gcg gag gtg ggt gag ttt ccg tgg cag gtg agt att     295
   Gly Gly Met Glu Ala Glu Val Gly Glu Phe Pro Trp Gln Val Ser Ile
    70                  75                  80                  85
   cag gca aga agt gaa cct ttc tgt ggc ggc tcc atc ctc aac aag tgg     343
   Gln Ala Arg Ser Glu Pro Phe Cys Gly Gly Ser Ile Leu Asn Lys Trp
                    90                  95                 100
   tgg att ctc act gcg gct cac tgc tta tat tcc gag gag ctg ttt cca     391
   Trp Ile Leu Thr Ala Ala His Cys Leu Tyr Ser Glu Glu Leu Phe Pro
               105                 110                 115
   gaa gaa ctg agt gtc gtg ctg ggg acc aac gac tta act agc cca tcc     439
   Glu Glu Leu Ser Val Val Leu Gly Thr Asn Asp Leu Thr Ser Pro Ser
           120                 125                 130
   atg gaa ata aag gag gtc gcc agc atc att ctt cac aaa gac ttt aag     487
   Met Glu Ile Lys Glu Val Ala Ser Ile Ile Leu His Lys Asp Phe Lys
       135                 140                 145
   aga gcc aac atg gac aat gac att gcc ttg ctg ctg ctg gct tcg ccc     535
   Arg Ala Asn Met Asp Asn Asp Ile Ala Leu Leu Leu Leu Ala Ser Pro
   150                 155                 160                 165
   atc aag ctc gat gac ctg aag gtg ccc atc tgc ctc ccc acg cag ccc     583
   Ile Lys Leu Asp Asp Leu Lys Val Pro Ile Cys Leu Pro Thr Gln Pro
                   170                 175                 180
   ggc cct gcc aca tgg cgc gaa tgc tgg gtg gca ggt tgg ggc cag acc     631
   Gly Pro Ala Thr Trp Arg Glu Cys Trp Val Ala Gly Trp Gly Gln Thr
               185                 190                 195
   aat gct gct gac aaa aac tct gtg aaa acg gat ctg atg aaa gcg cca     679
   Asn Ala Ala Asp Lys Asn Ser Val Lys Thr Asp Leu Met Lys Ala Pro
           200                 205                 210
   atg gtc atc atg gac tgg gag gag tgt tca aag atg ttt cca aaa ctt     727
   Met Val Ile Met Asp Trp Glu Glu Cys Ser Lys Met Phe Pro Lys Leu
       215                 220                 225
   acc aaa aat atg ctg tgt gcc gga tac aag aat gag agc tat gat gcc     775
   Thr Lys Asn Met Leu Cys Ala Gly Tyr Lys Asn Glu Ser Tyr Asp Ala
   230                 235                 240                 245
   tgc aag ggt gac agt ggg ggg cct ctg gtc tgc acc cca gag cct ggt     823
   Cys Lys Gly Asp Ser Gly Gly Pro Leu Val Cys Thr Pro Glu Pro Gly
                   250                 255                 260
   gag aag tgg tac cag gtg ggc atc atc agc tgg gga aag agc tgt gga     871
   Glu Lys Trp Tyr Gln Val Gly Ile Ile Ser Trp Gly Lys Ser Cys Gly
               265                 270                 275
   gat aag aac acc cca ggg ata tac acc tcg ttg gtg aac tac aac ctc     919
   Asp Lys Asn Thr Pro Gly Ile Tyr Thr Ser Leu Val Asn Tyr Asn Leu
           280                 285                 290
   tgg atc gag aaa gtg acc cag cta gga ggc agg ccc ttc aat gca gag     967
   Trp Ile Glu Lys Val Thr Gln Leu Gly Gly Arg Pro Phe Asn Ala Glu
       295                 300                 305
   aaa agg agg act tct gtc aaa cag aaa cct atg ggc tcc cca gtc tcg    1015
   Lys Arg Arg Thr Ser Val Lys Gln Lys Pro Met Gly Ser Pro Val Ser
   310                 315                 320                 325
   gga gtc cca gag cca ggc agc ccc aga tcc tgg ctc ctg ctc tgt ccc    1063
   Gly Val Pro Glu Pro Gly Ser Pro Arg Ser Trp Leu Leu Leu Cys Pro
                   330                 335                 340
   ctg tcc cat gtg ttg ttc aga gct att ttg tac tgataataaa atagaggcta 1116
   Leu Ser His Val Leu Phe Arg Ala Ile Leu Tyr
               345                 350
   ttctttc                                                            1123
<210> 10
   <211> 352
   <212> PRT
   <213> Homo sapiens
<400> 10
   Met Leu Leu Phe Ser
   1               5
   Val Leu Leu Leu Leu Ser Leu Val Thr Gly Thr Gln Leu Gly Pro Arg
                   10                  15                  20
   Thr Pro Leu Pro Glu Ala Gly Val Ala Ile Leu Gly Arg Ala Arg Gly
               25                  30                  35
   Ala His Arg Pro Gln Pro Arg His Pro Pro Ser Pro Val Ser Glu Cys
           40                  45                  50
   Gly Asp Arg Ser Ile Phe Glu Gly Arg Thr Arg Tyr Ser Arg Ile Thr
        55                  60                  65
   Gly Gly Met Glu Ala Glu Val Gly Glu Phe Pro Trp Gln Val Ser Ile
    70                  75                  80                  85
   Gln Ala Arg Ser Glu Pro Phe Cys Gly Gly Ser Ile Leu Asn Lys Trp
                    90                  95                  100
   Trp Ile Leu Thr Ala Ala His Cys Leu Tyr Ser Glu Glu Leu Phe Pro
               105                 110                 115
   Glu Glu Leu Ser Val Val Leu Gly Thr Asn Asp Leu Thr Ser Pro Ser
           120                 125                 130
   Met Glu Ile Lys Glu Val Ala Ser Ile Ile Leu His Lys Asp Phe Lys
       135                 140                 145
   Arg Ala Asn Met Asp Asn Asp Ile Ala Leu Leu Leu Leu Ala Ser Pro
   150                 155                 160                 165
   Ile Lys Leu Asp Asp Leu Lys Val Pro Ile Cys Leu Pro Thr Gln Pro
                   170                 175                 180
   Gly Pro Ala Thr Trp Arg Glu Cys Trp Val Ala Gly Trp Gly Gln Thr
               185                 190                 195
   Asn Ala Ala Asp Lys Asn Ser Val Lys Thr Asp Leu Met Lys Ala Pro
           200                 205                 210
   Met Val Ile Met Asp Trp Glu Glu Cys Ser Lys Met Phe Pro Lys Leu
       215                 220                 225
   Thr Lys Asn Met Leu Cys Ala Gly Tyr Lys Asn Glu Ser Tyr Asp Ala
   230                 235                 240                 245
   Cys Lys Gly Asp Ser Gly Gly Pro Leu Val Cys Thr Pro Glu Pro Gly
                   250                 255                 260
   Glu Lys Trp Tyr Gln Val Gly Ile Ile Ser Trp Gly Lys Ser Cys Gly
               265                 270                 275
   Asp Lys Asn Thr Pro Gly Ile Tyr Thr Ser Leu Val Asn Tyr Asn Leu
           280                 285                 290
   Trp Ile Glu Lys Val Thr Gln Leu Gly Gly Arg Pro Phe Asn Ala Glu
       295                 300                 305
   Lys Arg Arg Thr Ser Val Lys Gln Lys Pro Met Gly Ser Pro Val Ser
   310                 315                 320                 325
   Gly Val Pro Glu Pro Gly Ser Pro Arg Ser Trp Leu Leu Leu Cys Pro
                   330                 335                 340
   Leu Ser His Val Leu Phe Arg Ala Ile Leu Tyr
               345                 350
<210> 11
   <211> 22
   <212> DNA
   <213> Artificial Sequence
<220>
   <221> Source
   <223> Description of Artificial Sequence: "76A5sc2-B", an artificially synthesized primer sequence.
<400> 11
   gatcmacagg tgccagtcat ca 22
<210> 12
   <211> 20
   <212> DNA
   <213> Artificial Sequence
<220>
   <221> Source
   <223> Description of Artificial Sequence: "SPORT SP6", an artificially synthesized primer sequence.
<400> 12
   atttaggtga cactatagaa 20
<210> 13
   <211> 18
   <212> DNA
   <213> Artificial Sequence
<220>
   <221> Source
   <223> Description of Artificial Sequence: "SPORT FW", an artificially synthesized primer sequence.
<400> 13
   tgtaaaacga cggccagt 18
<210> 14
   <211> 18
   <212> DNA
   <213> Artificial Sequence
<220> .
   <221> Source
   <223> Description of Artificial Sequence: "SPORT RV", an artificially synthesized primer sequence.
<400> 14
   caggaaacag ctatgacc 18
<210> 15
   <211> 22
   <212> DNA
   <213> Artificial Sequence
<220>
   <221> Source
   <223> Description of Artificial Sequence: "No9-C", an artificially synthesized primer sequence.
<400> 15
   atgcttctgc tatcgtggaa gg 22
<210> 16
   <211> 20
   <212> DNA
   <213> Artificial Sequence
<220>
   <221> Source
   <223> Description of Artificial Sequence: "SPORT T7", an artificially synthesized primer sequence.
<400> 16
   taatacgact cactataggg 20
<210> 17
   <211> 22
   <212> DNA
   <213> Artificial Sequence
<220>
   <221> Source
   <223> Description of Artificial Sequence: "No9-B", an artificially synthesized primer sequence.
<400> 17
   ctttgtgctg aggtcttcag tg 22
<210> 18
   <211> 22
   <212> DNA
   <213> Artificial Sequence
<220>
   <221> Source
   <223> Description of Artificial Sequence: "No9-G", an artificially synthesized primer sequence.
<400> 18
   cagtcaatgt cactgtggtc at 22
<210> 19
   <211> 22
   <212> DNA
   <213> Artificial Sequence
<220>
   <221> Source
   <223> Description of Artificial Sequence: "No9-J", an artificially synthesized primer sequence.
<400> 19
   acttgccgtt ggtgcccact tc 22
<210> 20
   <211> 23
   <212> DNA
   <213> Artificial Sequence
<220>
   <221> Source
   <223> Description of Artificial Sequence: "No9-P", an artificially synthesized primer sequence.
<400> 20
   gcactggaat gacaacatga tgc 23
<210> 21
   <211> 22
   <212> DNA
   <213> Artificial Sequence
<220>
   <221> Source
   <223> Description of Artificial Sequence: "No9-Q", an artificially synthesized primer sequence.
<400> 21
   attggcgtgg caagtaggag ca 22
<210> 22
   <211> 22
   <212> DNA
   <213> Artificial Sequence
<220>
   <221> Source
   <223> Description of Artificial Sequence: "No9-N", an artificially synthesized primer sequence.
<400> 22
   cgagtctccc agttagcaca ga 22
<210> 23
   <211> 22
   <212> DNA
   <213> Artificial Sequence
<220>
   <221> Source
   <223> Description of Artificial Sequence: "No9-M' ", an artificially synthesized primer sequence.
<400> 23
   cggtgacttg gtcatgtctg tg 22
<210> 24
   <211> 29
   <212> DNA
   <213> Artificial Sequence
<220>
   <221> Source
   <223> Description of Artificial Sequence: "No9-K", an artificially synthesized primer sequence.
<400> 24
   ggatccatga aacgatggaa ggacagaag 29
<210> 25
   <211> 22
   <212> DNA
   <213> Artificial Sequence
<220>
   <221> Source
   <223> Description of Artificial Sequence: "No9-O" an artificially synthesized primer sequence.
<400> 25
   cgcagagttc tgctcataca ta 22
<210> 26
   <211> 21
   <212> DNA
   <213> Artificial Sequence
<220>
   <221> Source
   <223> Description of Artificial Sequence: "No9-A", an artificially synthesized primer sequence.
<400> 26
   ggcatgtagc tcactggcat g 21
<210> 27
   <211> 22
   <212> DNA
   <213> Artificial Sequence
<220>
   <221> Source
   <223> Description of Artificial Sequence: "29(-)", an artificially synthesized primer sequence.
<400> 27
   ggaccagcaa gaatcagttc tg 22
<210> 28
   <211> 22
   <212> DNA
   <213> Artificial Sequence
<220>
   <221> Source
   <223> Description of Artificial Sequence: "17(+)95(+)", an artificially synthesized primer sequence.
<400> 28
   ctgctaccag ttctaatttg cc 22
<210> 29
   <211> 22
   <212> DNA
   <213> Artificial Sequence
<220>
   <221> Source
   <223> Description of Artificial Sequence: "G3PDH 5' ", an artificially synthesized primer sequence.
<400> 29
   gagattgttg ccatcaacga cc 22
<210> 30
   <211> 22
   <212> DNA
   <213> Artificial Sequence
<220>
   <221> Source
   <223> Description of Artificial Sequence: "G3PDH 3' ", an artificially synthesized primer sequence.
<400> 30
   gttgaagtcg caggagacaa cc 22
<210> 31
   <211> 21
   <212> DNA
   <213> Artificial Sequence
<220>
   <221> Source
   <223> Description of Artificial Sequence: "h-B", an artificially synthesized primer sequence.
<400> 31
   agaggtcact gtcgagctgg g 21
<210> 32
   <211> 22
   <212> DNA
   <213> Artificial Sequence
<220>
   <221> Source
   <223> Description of Artificial Sequence: "h-D", an artificially synthesized primer sequence.
<400> 32
   tgtgaataat gaccttctgc ac 22
<210> 33
   <211> 22
   <212> DNA
   <213> Artificial Sequence
<220>
   <221> Source
   <223> Description of Artificial Sequence: "h-A ", an artificially synthesized primer sequence.
<400> 33
   ttcagcaaca tccactcgga ga 22
<210> 34
   <211> 22
   <212> DNA
   <213> Artificial Sequence
<220>
   <221> Source
   <223> Description of Artificial Sequence: "h-C", an artificially synthesized primer sequence.
<400> 34
   aagcaagtgc agaaggtcat ta 22
<210> 35
   <211> 22
   <212> DNA
   <213> Artificial Sequence
<220>
   <221> Source
   <223> Description of Artificial Sequence: "h-F", an artificially synthesized primer sequence.
<400> 35
   cattggtcgt tacccactgt gc 22
<210> 36
   <211> 23
   <212> DNA
   <213> Artificial Sequence
<220>
   <221> Source
   <223> Description of Artificial Sequence: "PRO1-E", an artificially synthesized primer sequence.
<400> 36
   attctcaatg agtggtgggt tct 23
<210> 37
   <211> 22
   <212> DNA
   <213> Artificial Sequence
<220>
   <221> Source
   <223> Description of Artificial Sequence: "PR01-D", an artificially synthesized primer sequence.
<400> 37
   ccagcacaca gcatattctt gg 22
<210> 38
   <211> 25
   <212> DNA
   <213> Artificial Sequence
<220>
   <221> Source
   <223> Description of Artificial Sequence: "hPR03-B", an artificially synthesized primer sequence.
<400> 38
   ggaaacagct cctcggaata taagc 25
<210> 39
   <211> 25
   <212> DNA
   <213> Artificial Sequence
<220>
   <221> Source
   <223> Description of Artificial Sequence: "hPR03-D", an artificially synthesized primer sequence.
<400> 39
   tggatgggct agttaagtcg ttggt 25
<210> 40
   <211> 23
   <212> DNA
   <213> Artificial Sequence
<220>
   <221> Source
   <223> Description of Artificial Sequence: "hPR03-A", an artificially synthesized primer sequence.
<400> 40
   ttcgagggaa gaactcggta ttc 23
<210> 41
   <211> 25
   <212> DNA
   <213> Artificial Sequence
<220>
   <221> Source
   <223> Description of Artificial Sequence: "hPR03-C", an artificially synthesized primer sequence.
<400> 41
   tgtgaaaacg gatctgatga aagcg 25
<210> 42
   <211> 22
   <212> DNA
   <213> Artificial Sequence
<220>
   <221> Source
   <223> Description of Artificial Sequence: "mPR03-B", an artificially synthesized primer sequence.
<400> 42
   cacctactgc caggatctgt gg 22
<210> 43
   <211> 25
   <212> DNA
   <213> Artificial Sequence
<220>
   <221> Source
   <223> Description of Artificial Sequence: "mPR03-D", an artificially synthesized primer sequence.
<400> 43
   ggctattttc tcaatccaca gggta 25
<210> 44
   <211> 25
   <212> DNA
   <213> Artificial Sequence
<220>
   <221> Source
   <223> Description of Artificial Sequence: "mPR03-A", an artificially synthesized primer sequence.
<400> 44
   atagagtggg aggaatgctt acaga 25
<210> 45
   <211> 25
   <212> DNA
   <213> Artificial Sequence
<220>
   <221> Source
   <223> Description of Artificial Sequence: "mPR03-C", an artificially synthesized primer sequence.
<400> 45
   gctacgatgc ttgccagggt g 21

## Description

### Technical Field

The present invention relates to novel trypsin-family serine proteases, the genes encoding them, and the production and uses thereof.

### Background Art

In the testis, the male reproductive organ, sperm, i.e. male gametes, are primarily formed through the following three-step process: (1)' the self-reproduction of spermatogonium as the germ-line stem cell and the initiation of differentiation thereof to the sperm, (2) meiotic division of spermatocyte and the associated gene recombination, and (3) morphogenesis of the haploid spermatid to the sperm. The sperms formed in this manner are expelled into a female body by coitus, pass along the oviduct, and bind to an egg, the female gamete, to achieve fertilization (Yomogida, K. and Nishimune, Y. (1998) Protein, Nucleic acid and Enzyme, 511-521) . To achieve fertilization, it is necessary for a sperm to move through the oviduct, adhere to and penetrate the zona pellucida on the egg surface, and then fuse with the egg.

A variety of proteases participate in these steps of the fertilization process. For example, an analysis using knockout mice (Krege, J.H. et al. (1995) Nature 375: 146-148; Esther Jr, C.R. et al. (1996) Lab. Invest. 74: 953-965) has revealed that sperm angiotensin-converting enzyme (testis ACE) plays an important role in the process of sperm transportation within the oviduct (Hagaman, J.R. et al. (1998) Proc. Natl. Acad. Sci. USA 95: 2552-2557). Fertilizing ability is markedly reduced in the male knockout mice that lack proprotein convertase 4 (PC4) (M. Mbikay et al. (1997) Proc.' Natl. Acad. Sci. USA, 94: 6842-6846).

Regarding serine proteases, a variety of trypsin inhibitors inhibit in vitro fertilization, suggesting that trypsin-like serine proteases present in the sperm (the acrosome in particular) may digest the zona pellucida when the sperm penetrates the zona pellucida (Saling, P.M. (1981) Proc. Natl. Acad. Sci. USA, 78: 6231-6235; Benau, D.A. and Storey, B.T. (1987) Biol. Reprod., 36: 282-292; Liu D.Y. and Baker, H.W. (1993) Biol. Reprod., 48: 340-348). Previously, acrosin, a trypsin-family serine protease in the acrosome, was assumed to play this role (Brown, C.R. (1983) J. Reprod. Fertil., 69: 289-295; Kremling, H. et al. (1991) Genomics, 11: 828-834; Klemm, U. et al., (1990) Differentiation, 42: 160-166). However, acrosin knockout mice have been shown to have almost normal fertilizing ability, suggesting that other serine proteases which are present in the sperm, apart from acrosin, digest zona pellucida (Baba, T. et al. (1994) J. Biol. Chem., 269: 31845-31849; Adham, I.M. et al. (1997) Mol. Reprod. Dev., 46: 370-376). In ascidians, a trypsin-family serine protease, called spermosin, is expressed in the sperm (Sawada, H. et al. (1984) J. Biol. Chem., 259: 2900-2904) . An antibody specific to this protease has been shown to inhibit fertilization in ascidians in a concentration-dependent manner (Sawada, H. et al., (1996) Biochem. Biophys. Res. Commun., 222: 499-504). Recently, cDNAs of the trypsin-family serine proteases, TESP1 and TESP2, which are expressed specifically in mouse acrosome, were cloned (Kohno, N. et al., (1998) Biochem. Biophys. Res. Commun., 245: 658-665). However, the roles these genes play in the fertilization process remains to be clarified. Moreover, serine proteases existing in the sperm and capable of digesting the zona pellucida have not yet been reported.

### Disclosure of the Invention

An objective of the present invention is to provide novel trypsin-family serine proteases associated with spermatogenesis and sperm functions, the genes encoding these proteases and a production method and use thereof.

The present inventors attempted to amplify a gene designated as 76A5sc2 by polymerase chain reaction, and eventually found a gene fragment having a nucleotide sequence different from that of 76A5sc2 gene. Using this gene fragment, the present inventors have cloned the cDNAs containing entire open reading frames (ORF) of two novel trypsin-family serine proteases ("Tespec PRO-1" and "Tespec PRO-2") expressed specifically in adult mouse testis. They have also analyzed the tissue-specific expression of these genes.

"Tespec PRO-1" (Testis specific expressed serine proteinase-1) is predicted to encode 321 amino acids. The deduced amino acid sequence contains trypsin-family serine protease motifs, "Trypsin-His" and "Trypsin-Ser" active sites, and exhibits significantly high homology to other trypsin-family serine proteases, such as acrosin, prostasin, trypsin and so on, in the regions of the two motifs and their neighboring regions. In the other regions, however, there are no known genes found to exhibit significant homology to this protein at the nucleotide or amino acid level. The foregoing demonstrates that this protein is a novel trypsin-family seine protease.

On the other hand, "Tespec PRO-2" is predicted to encode 319 amino acids. The protein has a "Trypsin-His" active site. With regard to the "Trypsin-Ser" active site, which consists of 12 amino acids, it is differs from that of the canonical motif by two amino acid residues. Such a difference is found in some other known trypsin-family serine proteases, and, thus, "Tespec PRO-2" is predicted to function as a protease. There are no known genes found to exhibit significant homology to "Tespec PRO-2" at the nucleotide and amino acid levels. Thus this protein is also a novel trypsin-family serine protease.

Interestingly, for "Tespec PRO-2", a splicing isoform was found that comprises the first half region of "Tespec PRO-2" connected to the latter half region of "Tespec PRO-1". This suggests that these two proteases are located very close to each other on the chromosome. Though a variety of splicing isoforms are found for "Tespec PRO-2", these "Tespec PRO-2" isoforms do not retain a long stretch of ORF, and thus do not encode any proteases at all. The homology between "Tespec PRO-1" and "Tespec PRO-2" is 52.2% at the nucleotide level and 33.1% at the amino acid level.

The present inventors have also successfully cloned a cDNA for human "Tespec PRO-2" by RT-PCR and RACE, based on the nucleotide sequence of mouse "Tespec PRO-2". Human "Tespec PRO-2" has been revealed to have 74.2% and 69.8% homology with mouse "Tespec PRO-2" at the nucleotide and amino acid levels, respectively. Further it has been clarified that human "Tespec PRO-2" is encoded on chromosome 8.

The present inventors have further succeeded in cloning a cDNA encoding human "Tespec PRO-3" by RT-PCR and RACE, based on the nucleotide sequence of mouse "Tespec PRO-1". In addition, they also succeeded in cloning a cDNA that encodes mouse "Tespec PRO-3", a mouse counterpart to human "Tespec PRO-3".

Northern blot analysis using the coding region for "Tespec PRO-1" as a probe revealed that this gene is expressed merely in adult mouse testis, but it failed to identify the expression in other tissues or in the fetal stage. Likewise, RT-PCR analysis also showed that expression of "Tespec PRO-1" is distinctly high in the adult testis. In addition, "Tespec PRO-1" was verified to have increased expression in the testis of 18 day-old mice or older, but it was not expressed in the testis of 12 day-old mice or younger or in the spermatogenesis-defect mutant mice. Similar analysis was carried out for "Tespec PRO-2" and revealed that expression pattern of this gene is identical to that of "Tespec PRO-1". These findings suggest that both "Tespec PRO-1" and "Tespec PRO-2" are involved in sperm differentiation and maturation, and/or sperm function (fertilization). It should be noted that trypsin-family serine proteases have been suggested to play important roles in fertilization.

Thus, the present inventors conclude that the proteins encoded by the isolated genes are likely serine proteases that play crucial roles in fertilization. Accordingly, they may be useful for developing new therapeutic or diagnostic agents for sterility, and/or for developing new contraceptives.

The present invention relates to novel trypsin-family serine proteases thought to be associated with spermatogenesis or sperm functions, the genes encoding them, production methods and the uses thereof. More specifically, the present invention provides:
1. a protein comprising the amino acid sequence selected from the group consisting of SEQ ID NO: 2, SEQ ID NO: 4, SEQ ID NO: 6, SEQ ID NO: 8, and SEQ ID NO: 10;
2. a protein functionally equivalent to the protein comprising an amino acid sequence selected from the group consisting of SEQ ID NO: 2, SEQ ID NO: 4, SEQ ID NO: 6, SEQ ID NO: 8, and SEQ ID NO: 10, wherein said protein is selected from the group of (a) and (b), wherein:
   (a) is a protein comprising an amino acid sequence selected from the group consisting of SEQ ID NO: 2, SEQ ID NO: 4, SEQ ID NO: 6, SEQ ID NO: 8, and SEQ ID NO: 10, wherein one or more amino acids are deleted, added, inserted and/or substituted with different amino acids; and
   (b) is a protein encoded by DNA that hybridizes to the DNA comprising the nucleotide sequence selected from the group consisting of SEQ ID NO: 1, SEQ ID NO: 3, SEQ ID NO: 5, SEQ ID NO: 7, and SEQ ID NO: 9;
3. a partial peptide of the protein according to any one of (1) and (2) ;
4. a fusion protein comprising the first protein according to any one of (1) and (2), fused with a second peptide;
5. a DNA molecule encoding the protein according to any one of (1) to (3) ;
6. a vector into which the DNA according to (5) is inserted;
7. a transformant having the DNA according to (5) in an expressible form;
8. a method for producing the protein according to any one of (1) to (3), said method comprising the steps of: culturing the transformant according to (7), and recovering the expressed protein from the transformant or the culture supernatant thereof;
9. a method of screening for a substrate of the protein according to any of (1) and (2), wherein the method comprises the following steps of:
   (a) contacting a test sample with said protein;
   (b) detecting the protease activity of said protein against the test sample; and
   (c) selecting a compound that is digested or cleaved by said protease activity;
10. a substrate of the protein according to any of (1) and (2), wherein said substrate can be isolated by the method according to (9) ;
11. a method of screening for a compound capable of inhibiting the activity of the protein according to any of (1) and (2), said method comprising the following steps of:
   (a) contacting the protein with the substrate of (10) in the presence of a test sample;
   (b) detecting the protease activity of the protein against the substrate; and
   (c) selecting a compound that reduces the protease activity relative to the protease activity detected in the absence of the test sample;
12. a compound that inhibits the activity of the protein according to any of (1) and (2), wherein said compound can be isolated by the method according to (11);
13. an antibody that binds to the protein according to any of (1) and (2);
14. a method for detecting or assaying the protein according to any of (1) and (2), said method comprising the steps of: contacting the antibody according to (13) with a test sample that is anticipated to contain the protein; and detecting or assaying formation of the immune-complex between the antibody and the protein; and
15. a nucleotide sequence specifically hybridizing to the DNA comprising the nucleotide sequence selected from the group consisting of SEQ ID NO: 1, SEQ ID NO: 3, SEQ ID NO: 5, SEQ ID NO: 7, and SEQ ID NO: 9, wherein the nucleotide sequence is at least 15 nucleotide in length.

The present invention provides novel trypsin-family serine proteases. Of the proteins provided in the present invention, the amino acid sequence of the mouse protein designated "Tespec PRO-1" is shown in SEQ ID NO: 2, the amino acid sequences of the mouse and human proteins designated "Tespec PRO-2" are shown in SEQ ID NO: 4 and SEQ ID NO: 6, respectively, and the amino acid sequences of the mouse and human proteins designated "Tespec PRO-3" are shown in SEQ ID NO: 8 and SEQ ID NO: 10, respectively. Nucleotide sequences of the cDNA encoding these proteins are shown in SEQ ID NO: 1, SEQ ID NO: 3, SEQ ID NO: 5, SEQ ID NO: 7, and SEQ ID NO: 9, respectively.

A high level of expression of the proteins of the present invention "Tespec PRO-1" and "Tespec Pyro-2" were observed in the mouse testis (Examples 5 and 6) . When these proteins are localized in the sperm, particularly in the acrosome region, they may function as key proteases for sperm to achieve fertilization by digesting the zona pellucida. Thus, the proteins of the present invention may be useful for developing new therapeutic and diagnostic agents for sterility or for developing new contraceptives.

The present invention also encompasses proteins that are functionally equivalent to mouse "Tespec PRO-1", mouse "Tespec PRO-2", human "Tespec PRO-2", mouse "Tespec PRO-3", or human "Tespec PRO-3" protein. As used herein, the term "functionally equivalent" refers to the retention of biological properties equivalent to mouse "Tespec PRO-1", mouse "Tespec PRO-2", human "Tespec PRO-2", mouse "Tespec PRO-3", or human "Tespec PRO-3" protein. Illustrative biological properties include, but are not limited to, for example, (i) trypsin-family serine protease activity as an activity property, (ii) trypsin-family serine protease motifs ("Trypsin-His" (PROSITE PS00134), "Trypsin-Ser" (PROSITE PS00135)) and/or similar sequences thereof, as well as significant homology to the amino acid sequence of mouse "Tespec PRO-1" protein, mouse "Tespec PRO-2" protein, human "Tespec PRO-2" protein, mouse "Tespec PRO-3" proteins, or human "Tespec PRO-3" protein as the structural properties of the sequences (infra), and (iii)expression in the testis, as the expression property.

Methods for introducing mutations into the amino acid sequence of a protein, for example, may be used to obtain such functionally equivalent proteins. To obtain a protein into which mutations are introduced into its amino acid sequence, methods such as site-specific mutagenesis using synthetic oligonucleotide primers (Kramer, W. and Fritz, H. J. Methods in Enzymol., (1987) 154: 350-367), a PCR system for site-specific mutagenesis (GIBCO-BRL) and the Kunkel's method (Methods Enzymol., (1988) 85: 2763-2766) may be used. By these methods, a protein comprising the amino acid sequence of SEQ ID NO: 2, SEQ ID NO: 4, SEQ ID NO: 6, SEQ ID NO: 8, or SEQ ID NO: 10 can be modified to obtain a protein in which one or more amino acids in its amino acid sequence have been deleted, added, inserted and/or substituted with different amino acids without affecting the biological properties of the protein.

There is no particular limitation on the number of amino acids that may be mutagenized, as long as the protein retains the biological properties of the wild-type protein (comprising the amino acid sequence of SEQ ID NO: 2, SEQ ID NO: 4, SEQ ID NO: 6, SEQ ID NO: 8 or SEQ ID NO: 10). Such mutations include, but are not limited to, for example:
- deletion of one or more amino acids, preferably, 2 to 30, and more preferably, 2 to 10 amino acids from any one of the amino acid sequences of SEQ ID NO: 2, SEQ ID NO: 4, SEQ ID NO: 6, SEQ ID NO: 8, and SEQ ID NO: 10;
- addition of one or more amino acids, preferably, 2 to 30, and more preferably, 2 to 10 amino acids into any one of the amino acid sequences of SEQ ID NO: 2, SEQ ID NO: 4, SEQ ID NO: 6, SEQ ID NO: 8, and SEQ ID NO: 10; and
- substitution of one or more, preferably, 2 to 30, and more preferably, 2 to 10 amino acids in any one of the amino acid sequences of SEQ ID NO: 2, SEQ ID NO: 4, SEQ ID NO: 6, SEQ ID NO: 8, and SEQ ID NO: 10,with different amino acids.

There is also no particular limitation on the amino acid sites for mutagenesis, so long as the protein retains the biological properties of the wild-type protein comprising any one of the amino acid sequences shown in SEQ ID NOs: 2, 4, 6, 8 and 10.

It is known that a protein comprising a modified amino acid sequence of another protein wherein one or more amino acid residues have been deleted, added, and/or substituted with different amino acids can maintain its biological activity (Mark, D. F. et al. , Proc. Natl. Acad. Sci. USA, (1984) 81: 5662-5666; Zoller, M. J. & Smith, M., Nucleic Acids Research, (1982) 10: 6487-6500; Wang, A. et al., Science, 224: 1431-1433; Dalbadie-McFarland, G. et al., Proc. Natl. Acad. Sci. USA, (1982) 79: 6409-6413).

For example, proteins into which one or more amino acid residues have been added to proteins of the present invention include fusion proteins. A fusion protein is a protein made by fusing the protein of the present invention with another peptide. A fusion protein can be prepared in an artificial manner. For example, the DNA encoding the protein of the present invention can be ligated in-frame with a DNA encoding another peptide, and then introduced into an expression vector to express the fusion gene in a host using conventional methods. There is no particular restriction on the other peptides or proteins to be used for fusion with the protein of the present invention. Such peptides include, but are not limited to, for example, FLAG (Hopp, T.P. et al., BioTechnology, (1988) 6: 1204-1210), 6x His consisting of six histidine (His) residues, 10x His, influenza virus hemagglutinin (HA), human c-myc fragments, VSV-GP fragments, p18HIV fragments, T7-tag, HSV-tag, E-tag, SV40T antigen fragments, lck tag, α-tubulin fragments, B-tag, Protein C fragment, and other well-known peptides. Such proteins include, for example, GST (glutathione-S-transferase), HA (influenza virus hemagglutinin), immunoglobulin constant regions, β-galactosidase, MBP (maltose-binding protein), etc. Commercially available DNAs encoding these peptides or proteins may also be used to prepare fusion proteins.

Using well-known hybridization techniques (Sambrook, J et al., Molecular Cloning 2nd ed., 9.47-9.58, Cold Spring Harbor Lab. Press, 1989) and the DNA encoding the proteins of the present invention (DNA sequences of SEQ ID NOs: 1, 3, 5, 7 and 9) or a part thereof, one skilled in the art can isolate DNA homologous to the original DNA. Using the DNA thus obtained, one skilled in the art can routinely to obtain a protein functionally equivalent to the protein of the present invention. The present invention includes proteins that are functionally equivalent to the proteins of the present invention, including those which are encoded by DNA capable of hybridizing to the DNA encoding any of the aforementioned proteins of the present invention, or a part thereof, under a stringent condition. In the isolation of such hybridizable DNA from other organisms, there is no limitation on the type of organisms; such organisms include, but are not limited to, for example, human, mouse, rat, cattle, monkey, pig, etc. In the context of the present invention, the term "stringent conditions" typically refers to "42°C, 2x SSC, 0.1% SDS" and the like, preferably "50°C, 2x SSC, 0.1% SDS" and the like, and more preferably "65°C, 2x SSC, 0.1% SDS" and the like. Under these conditions, the higher the temperature is set, the higher the likelihood that DNA with higher homology will be obtained.

Proteins encoded by DNA isolated by the above hybridization techniques normally have high homology to the amino acid sequence of SEQ ID NO: 2, SEQ ID NO: 4, SEQ ID NO: 6, SEQ ID NO: 8, or SEQ ID NO: 10. In the context of the present invention, the term "high homology" typically refers toat least 60% homology, preferably at least 70% homology, more preferably at least 80% homology, even more preferably at least 95%. The degree of homology between two proteins can be determined using the algorithm described in Wilbur, W.J. and Lipman, D.J. Proc. Natl. Acad. Sci. USA, (1983) 80: 726-730.

The proteins of the present invention may differ in amino acid sequence, molecular weight, isoelectric point, presence or absence of a sugar chain, and form, according to the cells or hosts producing the proteins, or to the purification methods. However, as long as the obtained proteins retain the biological properties of the proteins comprising the amino acid sequence of SEQ ID NO: 2, SEQ ID NO: 4, SEQ ID NO: 6, SEQ ID NO: 8 or SEQ ID NO: 10, they are included in the present invention.

The protein of the present invention can be a naturally occurring protein or can be produced as a recombinant protein, utilizing a genetic recombination technique. A naturally occurring protein can be prepared, for example, by extracting proteins from tissue or cells (for example, testis) in which the proteins of the present invention are thought to be present, and then by performing affinity chromatography using the antibodies of the present invention described below.

Likewise, for example, to produce a recombinant protein, DNA encoding the protein of the present invention is incorporated into an expression vector in a manner such that the DNA is expressed under the control of expression regulatory regions, such as enhancers and promoters, and then transduced into host cells to express the protein.

Specifically, when mammalian cells are used, DNA corresponding to a conventional, useful promoter/enhancer, DNA encoding a protein of the present invention, and the poly A signal at the downstream region of the 3' end of the coding region are functionally linked or constructed as a vector containing such DNA. Exemplary promoters/enhancers include, but are not limited to, human cytomegalovirus immediate early promoter/enhancer.

Other promoters/enhancers that can be used for protein expression include, but are not limited to, retroviral, polyomaviral, adenoviral and simian virus 40 (SV40) promoters/enhancers, and promoters/enhancers derived from mammalian cells, such as that of human elongation factor 1α (HEF1α).

This is easily carried out, for example, according to the method of Mulligan et al. (Nature (1979) 277: 108) when SV40 promoter/enhancer is used, and to the method of Mizushima et al. (Nucleic Acids Res. (1990) 18: 5322) when using HEF1α promoter/enhancer is used.

For a replication origin, those derived from SV40, polyomavirus, adenovirus, bovine papilomavirus (BPV), and the like may be used. To increase the copy number of the gene in the host cell, the expression vector may.optionally contain a selectable marker, such as an aminoglycoside transferase (APH), thymidine kinase (TK), E.coli xanthine-guanine phosphoribosyl transferase (Ecogpt), or dihydrofolate reductase (dhfr) gene, etc.

When using E. coli, conventional useful promoters, a signal sequence for polypeptide secretion, and the gene to be expressed may be functionally linked to express the gene. Such promoters include, but are not limited to, for example, lacZ and araB promoters. When the lacZ promoter is used, the method of Ward et al. (Nature (1098) 341: 544-546; FASEB J. (1992) 6: 2422-2427) can be used. When the araB promoter is used, the method of Better et al. (Science (1988) 240: 1041-1043) may be followed.

To produce the protein into the periplasm of E. coli, the prelB signal sequence (Lei, S. P. et al., J. Bacteriol., (1987) 169: 4379) may be used as a signal for secretion of the protein.

Any expression vector can be used to produce the protein of the present invention so long as it is suitable for use with the present invention. Such expression vectors include, but are not limited to, for example, the adenoviral vector "pAdexLcw" and the retroviral vector "pZIPneo". Also included are expression vectors derived from mammalians, including, but not limited to, for example, pEF and pCDM8; derived from insects, including, but not limited to, for example, pBacPAK8; derived from plants, including, but not limited to, for example, pMH1 and pMH2; derived from animal viruses, including, but not limited to, for example, pHSV, pMV, and pAdexLcw; derived from retroviruses, including, but not limited to, for example, pZIpneo; derived from yeast, including, but not limited to, for example, pNV11 and SP-Q01; derived from Bacillus subtilis, including, but not limited to, for example, pPL608 and pKTH50; and derived from E. coli, including, but not limited to, for example, pQE, pGEAPP, pGEMEAPP, pMALp2 and pREP4.

In the present invention, any production systems may be used to produce the protein. Such production systems for producing the protein include in vitro and in vivo production systems. Production systems using eukaryotic cells or prokaryotic cells may be used as in vitro production systems.

Among the production systems using eukaryotic cells are those using animal cells, plant cells, and fungal cells. Such animal cells include mammalian cells, such as CHO (J. Exp. Med. (1995) 108: 945), COS, myeloma, BHK (baby hamster kidney), HeLa, and Vero, ; amphibian cells, such as *Xenopus* oocytes (Valle, et al., Nature, (1981) 291: 358-340); insect cells, such as sf9, sf21 and Tn5. Particularly preferred are CHO cells, dhfr-CHO, a DHFR-deficient CHO cell (Proc. Natl. Acad. Sci. USA, (1980) 77 : 4216-4220), and CHO K-1 (Proc. Natl. Acad. Sci. USA, (1968) 60: 1275).

Nicotiana tabacum-derived cells are plant cells that are well known for such use. They can be grown as callus culture. As such fungal cells, yeasts, such as the *Saccharomyces* genus, for example, Saccharomyces cerevisiae, filamentous bacteria such as the *Aspergillus genus,* for example, *Aspergillus* niger are known.

Among the production systems using prokaryotic cells is a production system using bacterial cells. Such bacterial cells include E. coli and Bacillus subtilis.

These cells are transformed with.the DNA of interest, and the transformed cells are then cultured in vitro to obtain the proteins. The culture is performed according to conventional methods. For eukaryotic cells, culture media, such as DMEM, MEM, RPMI1640, and IMDM, can be used. These media may be used with a serum supplement, such as fetal calf serum (FCS), or used as a serum-free medium. Preferably pH of the culture ranges from about 6 to about 8. The culture is usually conducted for about 15 to 200 hours at a temperature of about 30°C to 40°C, and, if necessary, the medium may be changed, aerated, and stirred.

On the other hand, in vivo production systems include systems using animals and plants. The DNA of interest is introduced into such a plant or animal, within which the protein is produced, and then the protein produced is recovered. As used herein, the term "host" encompasses such animals and plants as well.

The systems using animals include the production systems using mammals and insects. Such mammals include, but are not limited to, goats, pigs, sheep, mice, and cattle (Vicki Glaser, SPECTRUM Biotechnology Applications, 1993). When mammals are used, transgenic animals may be used. For example, the DNA of interest is inserted within a gene encoding a protein produced intrinsically in milk, such as goat β casein, to prepare a fusion gene. The DNA fragment containing the fusion gene in which the DNA of interest is inserted injected into a goat embryo, which is then introduced into a female goat. The protein is then collected from the milk produced from the transgenic goat, that which was born from the goat that had accepted the embryo, or descendents thereof. To increase the amount of the milk containing the protein that is produced from the transgenic goat, suitable hormone(s) may be given to the transgenic goats (Ebert, K.M. et al., Bio/Technology, (1994) 12: 699-702).

Silk worms are useful insects in the context of the present invention. When a silk worm is used, it is infected with a baculovirus into which the DNA of interest has been inserted, and the desired protein is obtained from the body fluids of the silk worm (Susumu, M. et al., Nature, (1985) 315: 592-594).

When a plant is used, tobacco, for example, can be used. When a tobacco plant is used, the DNA of interest is inserted into a plant expression vector, for example pMON 530, which is then introduced into a bacterium such as *Agrobacterium tumefaciens.* This bacterium is used to infect the tobacco plant, for example *Nicotiana* tabacum, to obtain the desired polypeptide from its leaves (Julian, K.-C. Ma, et al., Eur. J. Immunol., (1994) 24: 131-138).

The protein of the present invention thus obtained can be isolated from inside or outside of the cells, or from hosts and purified as a substantially pure and homogenous protein. The separation and purification of the protein is not limited to any particular method, and can be done using conventional methods for separation and purification. For example, chromatography columns, filtration, ultrafiltration, salting out, solvent precipitation, solvent extraction, distillation, immunoprecipitation, SDS-polyacrylamide gel electrophoresis, isoelectric focusing, dialysis, recrystalization and the like may be suitably selected or combined to separate/purify the protein.

Such chromatographies include, but are not limited to, for example, affinity chromatography, ion exchange chromatography, hydrophobic chromatography, gel filtration, reversed-phase chromatography, adsorption chromatography, etc. (Strategies for Protein Purification and Characterization: A Laboratory, Course Manual. Ed Daniel R. Marshak et al., Cold Spring Harbor Laboratory Press, 1996). These chromatographies can be done by liquid chromatography, such as HPLC, FPLC, etc. The present invention encompasses the proteins highly purified by these purification methods.

Optionally, by treating with an appropriate modification enzyme before or after the proteins are purified, the proteins can be modified or their peptides can be partially removed. Such modification enzymes include, but are not limited to, trypsin, chymotrypsin, lysyl endopeptidase, protein kinase, and glucosidase.

The present invention also comprises partial peptides from the proteins of the present invention. Such peptides can be utilized, for example, as immunogens to give antibodies capable of binding to the proteins of the present invention. For this purpose, such peptides will contain at least 12 amino acid residues, and preferably, at least 20 amino acid residues. Partial peptides of the proteins of the present invention may be produced by genetic engineering techniques or using well-known methods for synthesizing peptides, or by cleaving the protein of the present invention with a suitable peptidase. To synthesize peptides, solid-phase synthesis and liquid-phase synthesis may be also used.

A protein of the present invention or a partial peptide thereof that is expressed in a host by using a genetic engineering technique can be isolated from the cells or extracellular materials and can be purified as a substantially pure and homogeneous protein. There is no limitation on the methods of isolation and purification of the protein; any of the generally used methods for protein purification may be used to isolate and purify the protein. Separation and purification of the protein can be achieved by properly selecting or combining methods including, but not limited to, for example, column chromatography, filtration, ultrafiltration, salting out, solvent precipitation, solvent extraction, distillation, immunoprecipitation, SDS-polyacrylamide gel electrophoresis, isoelectric focusing, dialysis, and recrystallization.

Such chromatographies include, but not limited to, for example, affinity chromatography, ion exchange chromatography, hydrophobic chromatography, gel filtration, reversed-phase chromatography, adsorption chromatography, etc. (Strategies for Protein Purification and Characterization: A Laboratory Course Manual. Ed Daniel R. Marshak et al. , Cold Spring Harbor Laboratory Press, 1996). These chromatographies can be done by liquid chromatography, such as HPLC, FPLC, etc. The present invention encompasses the proteins highly purified by these purification methods.

Optionally, by treating with an appropriate modification enzyme before or after the proteins are purified, the proteins can be modified or their peptides can be partially removed. Such modification enzymes include trypsin, chymotrypsin, lysyl endopeptidase, protein kinase, and glucosidase.

Further, the present invention provides for DNA encoding the proteins of the present invention mentioned above. The DNA of the present invention can be used not only to produce the proteins of the present invention in vivo and in vitro, but also for gene therapy of, for example, mammals (e.g., human). It is expected that the genes of the present invention, in particular, may be applied to the gene therapy of infertility. When used in the gene therapy, the DNA of the present invention is inserted into a vector and then administered to the target sites in the body. The method of administration may be ex vivo or in vivo. The vectors of the present invention include such vectors as used for gene therapy.

Genomic DNA or cDNA that encodes the protein of the present invention may be obtained by screening a genomic.library, a cDNA library or the like, using a hybridization technique well known to one skilled in the art.

By using the obtained DNA or cDNA fragment as a probe, and further by screening genomic or cDNA libraries, the genes can be obtained from other cells, tissues, organs, or species. Genomic and cDNA libraries may be prepared by, for example, the method of Sambrook, J. et al., Molecular Cloning, Cold Spring Harbor Laboratory Press (1989). Also, commercially available DNA libraries may be used.

By determining the nucleotide sequence of the obtained cDNA, the translatable region encoded by the cDNA can be identified to obtain the amino acid sequence of the protein of the present invention.

Specifically, this can be done as follows. First, mRNA is isolated from cells, tissue, or an organ expressing a protein of the present invention. To isolate mRNA, a well-known method, for example, guanidine ultracentrifugation (Chirgwin, J.M. et al., Biochemistry, (1979) 18: 5294-5299), the AGPC method (Chomczynski, P. and Sacchi, N., Anal. Biochem., (1987) 162: 156-159), is used to isolate total RNA, from which mRNA is purified using mRNA Purification Kit (Pharmacia), etc. QuickPrep mRNA Purification Kit (Pharmacia) can be used to prepare mRNA directly.

cDNA is synthesized from the obtained mRNA by reverse transcriptase. It can be synthesized using the AMV Reverse Transcriptase First-strand cDNA Synthesis Kit (SEIKAGAKU KOGYO), etc. Also, it may be synthesized and amplified with the probes set forth herein, according to the 5'-RACE method (Frohman, M.A. et al., Proc. Natl. Acad. Sci. USA, (1988) 85: 8998-9002; Belyavsky, A. et al., Nucleic Acids Res., (1989) 17: 2919-2932) using the 5'-Ampli FINDER RACE KIT (Clontech) and the polymerase chain reaction (PCR).

The DNA fragment of interest is prepared from the PCR product obtained and ligated with vector DNA. Recombinant vectors are thus created, and they are introduced into host cells, such as E.coli. Colonies are selected to prepare the desired recombinant vector. The nucleotide sequence of the DNA of interest may be verified by a known method, for example, the dideoxy nucleotide chain termination method.

The DNA of the present invention can be designed to have a sequence with higher expression efficiency, taking into account the codon used in the host for the expression (Grantham, R. et al., Nucleic Acids Research, (1981) 9: r43-r74). Also, the DNA of the present invention may be modified using commercially available kits or well-known methods. Such modification(s) include, but are not limited to, for example, digestion with restriction enzymes, insertion of synthetic oligonucleotides or suitable DNA fragments, addition of linkers, insertion of a start codon (ATG) and/or stop codon (TAA, TGA, or TAG).

The DNA of the present invention encompasses, for example, the DNA comprising the nucleotide sequence extending from A at nucleotide 48 to C at nucleotide 1010 of the nucleotide sequence set forth in SEQ ID NO: 1; the DNA comprising the nucleotide sequence extending from A at nucleotide 69 to C at nucleotide 1025 of the nucleotide sequence set forth in SEQ ID NO: 3; the DNA comprising the nucleotide sequence extending from A at nucleotide 73 to A at nucleotide 867 of the nucleotide sequence set forth in SEQ ID NO: 5; the DNA comprising the nucleotide sequence extending from A at nucleotide 38 to A at nucleotide 1000 of the nucleotide sequence set forth in SEQ ID NO: 7; and the DNA comprising the nucleotide sequence extending from A at nucleotide 41 to C at nucleotide 1096 of the nucleotide sequence set forth in SEQ ID NO: 9.

The DNA of the present invention further encompasses DNA that hybridizes under stringent conditions to the DNA of any of the nucleotide sequences of SEQ ID NO: 1, SEQ ID NO: 3, SEQ ID NO: 5, SEQ ID NO: 7, and SEQ ID NO: 9, so long as the hybridizing DNA also encodes a protein functionally equivalent to the protein of the present invention.

The "stringent conditions" are typically "42°C, 2x SSC, 0.1% SDS" and the like, preferably "50°C, 2x SSC, 0.1% SDS" and the like, and more preferably "65°C, 2x SSC, 0.1% SDS" and the like. Under these conditions, the higher the temperature is set, the higher the likelihood that DNA with higher homology will be obtained.

The hybridizable DNA mentioned above may be, for example, naturally occurring DNA (for example, cDNA and genomic DNA). For naturally occurring DNA, organisms used for isolation of DNA encoding the functionally equivalent protein include, but are not limited to, for example, human, mouse, rat, cattle, monkey, pig, etc. For example, in such animals, in a working example described herein, the DNA of the present invention was isolated using cDNA derived from a tissue (for example, testis) in which mRNA capable of hybridizing to cDNA encoding the protein of the present invention was detected. DNA encoding the proteins of the present invention may be cDNA or genomic DNA, as well as synthetic DNA.

The present invention also provides for a method of screening for substrates of the proteins of the present invention. In the context of the present invention, the term "substrate" of the proteins of the present invention refers to a compound that is decomposed or cleaved at a specific site upon the binding of a protein of the present invention.

The compounds to be used as substrates are not restricted to proteins. For example, trypsin and chymotrypsin are known to cleave not only proteins but also amide and ester bonds in the derivatives of peptidic compounds (Farmer, D.A. et al., J. Biol. Chem., (1975) 250: 7366-7371; del Castillo, L.M. et al., Biochim. Biophys. Acta., (1971) 235: 358-69). Thus, in the present invention, there is no limitation on the types of substrates so long as they are decomposed or cleaved at a specific site upon the binding of a protein of the present invention. Such substrates may be peptides, analogues or derivatives (peptidic compounds) thereof, or non-peptidic compounds.

The method of screening for the substrates of the present invention comprises the steps of: (a) contacting a test sample with any of the protein of the present invention, (b) detecting the protease activity of the protein of the present invention against the test sample, and (c) selecting a compound that is decomposed or cleaved by the protease activity of the protein of the present invention.

Test samples used for screening are those expected to contain the substrates for the protein of the present invention, including, but not limited to, for example, cell extracts, extracts from animal tissues, expressed products of a gene library, purified or crude proteins, peptides, peptidic analogues or derivatives, non-peptidic compounds, synthetic compounds, and naturally occurring compounds.

In the screening of the substrates capable of binding to the proteins of the present invention, for example, a test sample is mixed with a protein of the present invention, and the mixture is incubated. Subsequently, a change within the test sample (cleavage or decomposition) is assayed. For example, when the test sample is a protein, the test sample can be assayed directly, or after azidated or bound to a fluorescent substance, to detect its changes in UV spectrum (Beynon, R. J. and Bond, J. S., Proteolytic enzymes (1989) IRL Press, pp. 25-55) and HPLC (Maier M, et al., FEBS Lett., (1988) 232: 395-398; Gau W, et al. Adv. Exp. Med. Biol. (1983) 156: 483-494) before and after the reaction, thereby measuring the protease activity.

When the test sample is a peptide (or an analogue or derivative thereof), such peptide (or an analogue or derivative thereof) consisting of several amino acids (often, but not limited to, one to five amino acid residues) is mixed with a protein of the present invention, and incubated. Subsequently, changes within the test sample are assayed. For example, the test sample may be labeled with a fluorescent compound (MEC: Kawabata S. et al. (1988) Eur. J. Biochem. , 172: 17-25; AMC: Morita T. et al. (1977) J. Biochem. , (Tokyo). 82: 1495-1498; AFC: Garrett JR, et al. (1985) Histochem. J., 17:805-817, etc.) at the carboxyl terminus. Then the protease activity may be assayed being indexed by the spectral changes of the fluorescent compound upon the cleavage of the test sample. Screening methods utilizing other fluorescently labeled peptide substrates can be used (Beynon, R. J. and Bond, J. S., Proteolytic enzymes (1989) IRL Press, pp. 25-55; Gossrau, R., et al. (1984) Adv. Exp. Med. Biol., 167: 191-207; and Yu, J.X. et al., J. Biol. Chem., (1994) 269: 18843-18848).

In addition, the principle of the above-mentioned methods can be applied to the screening by using, as the test compounds, synthetic compounds, a bank of naturally occurring substances, a lambda phage peptide display library, pin peptide synthetic compounds, etc. Also, high-throughput screening is possible by utilizing a combinatorial chemistry techniques (Wrighton, N.C., Farrell, F.X., Chang, R, Kashyap, A.K., Barbone, F.P., Mulcahy, L.S., Johnson, D.L., Barrett, R.W., Jolliffe, L.K., Dower, W.J., "Small peptides as potent mimetics of the protein hormone erythropoietin", Science (UNITED STATES), Jul 26, 1996, 273, p458-64; Verdine, G.L., "The combinatorial chemistry of nature", Nature (ENGLAND), Nov 7, 1996, 384: 11-13; Hogan, J.C. Jr., "Directed combinatorial chemistry", Nature (ENGLAND), Nov 7, 1996, 384: 17-19).

Once substrates for the proteins of the present invention are isolated by using the screening method mentioned above, screening for inhibitors of the proteins of the present invention may then be conducted, the inhibitors being indexed by their inhibitory activity against the protease activity of the proteins of the present invention to the substrates. Thus the present invention also provides for a method of screening for compounds inhibiting the activity of the proteins of the present invention.

This method comprises the steps of: (a) contacting a protein of the present invention with its substrate in the presence of a test sample, (b) detecting protease activity of the protein of the present invention to the substrate, and (c) selecting a compound capable of lowering the protease activity relative to that detected in the absence of the test sample.

The proteins of the present invention useful for screening include authentic proteins, recombinant proteins, and partial peptides derived therefrom. Test samples useful for screening include, but are not limited to, cell culture supernatant, expression products of a gene library, peptides, peptide analogues or derivatives, purified or crude proteins (including antibodies), non-peptidic compounds, synthetic compounds, products from fermentation of microorganisms, extracts from marine organisms, plant extracts, cell extracts, extracts from animal tissues, etc.

Screening for inhibitors of the proteins of the present invention can be performed, for example, by using the systems as described in the following references (Beynon, R. J. and Bond, J. S., Proteolytic enzymes (1989), IRL Press, pp. 25-55; Maier, M. et al. (1988) FEBS Lett. 232: 395-398; Gau, W. et al. Adv. Exp. Med. Biol., (1983) 156: 483-494; Kawabata, S. et al. (1988) Eur. J. Biochem. 172: 17-25; Morita, T. et al. (1977) J. Biochem., (Tokyo) 82: 1495-1498; Garrett, J. R. et al. (1985) Histochem. J. 17: 805-817; Gossrau, R. et al. (1984) Adv. Exp. Med. Biol. 167: 191-207; Yu, J. X. et al., (1994) J. Biol. Chem. , 269: 18843-18848). Further, given that a peptide substrate is a lead compound, compounds that have resulted from modification or substitution of a part of the structure of the lead compound can be used as the test compounds in the screening for inhibitors of the proteins of the present invention (Okamoto, S. et al. (1993) Methods Enzymol., 222: 328-340).

As described above, expression patterns and such of the proteins of the present invention suggest that the proteins of the present invention may be involved in sperm differentiation and maturation, or sperm function (fertilization). Inhibitors that are isolated using the screening method of the invention can be utilized to analyze the involvement of the proteins of the present invention in fertilization. For example, the inhibitors of the proteins of the present invention may be used for in vitro analysis of fertilization (Y. Toyoda et al., 1971, Jpn. J. Anim. Reprod., 16: 147-151; Y. Kuribayashi et al. , 1996, Fertil. Steril. , 66: 1012-1017), which can subsequently be used to determine whether the inhibitors are capable of inhibiting fertilization or not. Such an inhibitor of a protein of the present invention that is capable of inhibiting fertilization finds potential utility as, for example, a new contraceptive.

The compounds obtained by the screening method of the present invention may find practical utility as drugs for treating humans and other mammals, such as mice, rats, guinea pigs, rabbits, chicken, cats, dogs, sheep, pigs, cattle, monkeys, sacred baboons, and chimpanzees, according to a conventional means.

For example, the drugs can be administered orally, in the form of tablets coated with sugar, if necessary, capsules, elixirs or microcapsules, or they can be administered parenterally, in the form of injections of sterile solutions of water or other pharmaceutically acceptable solutions, or suspensions. For example, a compound having the activity to bind to a protein of the present invention can be mixed with a physiologically acceptable carrier, flavoring agent, excipient, vehicle, preservative, stabilizer, and/or bonding agent in the form of a unit dose that is required for pharmaceutical implementations accepted in general. These active ingredients enable the preparations to be obtained in a suitable volume within the indicated volume range.

Examples of additives that can be mixed into tablets and capsules include, but are not limited to, binders, such as gelatin, corn starch, tragacanth gum, and arabic gum; excipients, such as crystalline cellulose; swelling agents, such as cornstarch, gelatin, and alginic acid; lubricants such as magnesium stearate; sweeteners such as sucrose, lactose, and saccharin; and flavoring agents such as peppermint, Gaultheria adenothrix oil, and cherry. When the unit dosage form is a capsule, a liquid carrier, such as oil, can also be included in the above additives. Sterile compositions for injections can be formulated by following standard drug implementations provided for dissolving or suspending active substances in such a vehicle as distilled water, or natural vegetable oils, such as sesame oil and coconut oil.

For example, physiological saline and isotonic liquids including glucose or other adjuvants, such as D-sorbitol, D-mannose, D-mannitol, and sodium chloride, can be used as aqueous solutions for injections. These can be used in conjunction with suitable solubilizers, including, but not limited to, alcohol, specifically ethanol, polyalcohols such as propylene glycol and polyethylene glycol, non-ionic surfactants, such as Polysorbate 80 (TM) and HCO-50.

Sesame oil or soybean oil can be used as an oleaginous liquid and may be used in conjunction with a solubilizer, such as benzyl benzoate and benzyl alcohol. In addition, such a liquid can be combined with a buffer, such as phosphate buffer and sodium acetate buffers; a pain-killer, such as benzalkonium chloride and procaine hydrochloride; a stabilizer, such as benzyl alcohol and phenol; and an anti-oxidant. The prepared injection is usually filled into a suitable ampoule.

Although the doses of the compounds that are obtained by the screening method of the present invention varies according to the symptoms, typically, an amount of about 0.1 to about 100 mg per day, preferably, about 1.0 to about 50 mg per day, and more preferably, about 1.0 to about 20 mg per day is administered orally to an adult (body weight 60 kg).

When administered parenterally, doses will differ, depending on the patient, target organ, symptoms and method of administration. The daily dose of, usually about 0.01 to about 30 mg, preferably about 0.1 to about 20 mg and more preferably about 0.1 to about 10 mg for an adult (body weight 60 kg) is advantageously administered by intravenous injection. For administration to other animals, the amount is converted to 60 kg of body-weight.

The present invention further provides antibodies capable of binding to a protein of the present invention. Such antibodies can be utilized for detection and purification of the protein of the present invention, as well as for in vitro analysis for fertilization. An antibody can be obtained as a monoclonal antibody or a polyclonal antibody by using a well-known method.

An antibody that specifically binds to a protein of the present invention can be prepared by using the protein of the present invention as a sensitizing antigen for immunization, according to a standard immunizing method, by fusing the immune cells obtained with any known parent cells, using a conventional method of cell fusion, and by screening for the cells producing an antibody, using a standard screening technique.

Specifically, a monoclonal or polyclonal antibody that specifically binds to the proteins of the present invention may be prepared as follows.

For example, the protein of the present invention that is used as a sensitizing antigen for obtaining the antibody is not restricted by the animal species from which it is derived, but is preferably a protein derived from mammals, for example, humans, mice, or rats, especially from humans. Proteins of human origin can be obtained based on the nucleotide sequence or amino acid sequence disclosed herein.

A protein to be used as a sensitizing antigen in the present invention may be a protein of the present invention or a partial peptide thereof. Partial peptides of a protein include, for example, amino (N) terminal fragments of the protein, and carboxyl (C) terminal fragments. In the context of the present invention, the term "antibody" of the present invention refers to an antibody that binds to the full-length protein or a fragment thereof.

A gene encoding a protein of the present invention or a fragment thereof is inserted into a well-known expression vector system; and the host cells described herein are transformed. Subsequently, the protein of interest or a fragment thereof is obtained from the host cells or the culture medium, using a well-known method, and used as a sensitizing antigen. Also, cells expressing the protein and lysate thereof, and a chemically synthesized protein of the present invention and a partial peptide thereof may be used as sensitizing antigens.

Mammals that can be immunized with the sensitizing antigens generally include, but are not limited to, Rodentia, Lagomorpha and Primates. To generate monoclonal antibodies, it is preferable to select a mammal by considering its compatibility with parent cells used for cell fusion.

Animals belonging to Rodentia include, but are not limited to, for example, mice, rats, hamsters, etc. Animals belonging to Lagomorpha include, but are not limited to, for example, rabbits, and Primates include, but are not limited to, for example, monkeys. Among monkeys, monkeys of the infraorder Catarrhini (Old World monkeys), for example, cynomolgus monkeys, rhesus monkeys, sacred baboons, chimpanzees, are used.

Any of a number of well-known methods may be used to immunize animals with a sensitizing antigen. For example, the sensitizing antigen is generally injected into mammals intraperitoneally or subcutaneously. Specifically, the sensitizing antigen is diluted or suspended with a buffer, such as physiological saline and phosphate-buffered saline (PBS), to be prepared in an appropriate amount, and, if desired, mixed with a suitable amount of a common adjuvant, such as Freund's complete adjuvant. The antigen thugs prepared may be emulsified and then injected into the mammal. Thereafter, the sensitizing antigen suitably mixed with Freund's incomplete adjuvant is preferably challenged several times at four to 21 day intervals. A suitable carrier can also be used when an animal is immunized with the sensitizing antigen. After the immunization, elevation of the level of the desired antibody in the serum antibody is confirmed by a conventional method.

To obtain polyclonal antibodies against the proteins of the invention, blood is removed from the mammal sensitized with the antigen after the level of the desired antibody is confirmed to increase in the serum. Serum may be isolated from the blood by any well-known method. The serum containing the polyclonal antibody may be used as the polyclonal antibody, and further, if necessary, the fraction containing the polyclonal antibody may be isolated from the serum.

To obtain monoclonal antibodies, after verifying that the level of the desired antibody has been increased in the serum of the mammal sensitized with the above-described antigen, immunocytes are taken out from the mammal and used for cell fusion. In this procedure, preferable immunocytes for cell fusion are splenocytes in particular. Parent cells to be fused with the above immunocytes are preferably mammalian myeloma cells.

Cell fusion of the above immunocytes and myeloma cells may be routinely carried out using any well-known method, for example, the method of Milstein et al. (Galfre, G. and Milstein, C., Methods Enzymol., (1981) 73: 3-46).

Hybridomas obtained from the cell fusion are screened for selection by culturing them in a usual selective culture medium, for example, HAT culture medium (a medium containing hypoxanthine, aminopterin and thymidine). The culture in the HAT medium is continued for a sufficient period to eliminate the cells (non-fusion cells) except for the hybridomas of interest, usually for a few days to a few weeks. Subsequently, conventional limiting dilution analysis is performed to screen for and clone the hybridoma producing the antibody of interest.

In addition to obtaining the hybridomas mentioned above, by immunizing an animal other than human with the antigen, human lymphocytes, for example, human lymphocytes infected with EB virus, can be sensitized in *vitro* with a protein, protein-expressing cells or lysates thereof, and the sensitized lymphocytes can then be fused with myeloma cells derived from human that have the capacity of permanent cell division, for example U266, to obtain a hybridoma producing the human antibody of interest that comprises the binding activity to the protein (Unexamined Published Japanese Patent Application (JP-A) No. Sho 63-17688).

Moreover, a transgenic animal having a human antibody gene repertoire is immunized with an antigen, such as a protein, protein-expressing cells and cell lysate thereof to obtain antibody-producing cells, which are then fused with myeloma cells to obtain hybridomas. The hybridomas may be used to obtain a human antibody against the protein (WO92/03918 WO93/2227, WO94/02602, WO94/25585 WO96/33735 and WO96/34096).

Instead of producing antibodies from hybridomas, antibody-producing immunocytes such as sensitized lymphocytes that are immortalized with an oncogene may be used.

Such monoclonal antibodies, obtained as described above, can be produced as recombinant antibodies using genetic engineering techniques (for example, see Borrebaeck, C.A.K. and Larrick, J.W., THERAPEUTIC MONOCLONAL ANTIBODIES, Published in the United Kingdom by MACMILLAN PUBLISHERS LTD, 1990). A recombinant antibody may be produced as follows: the DNA encoding the antibody is cloned from a hybridoma or immunocytes, such as sensitized lymphocytes producing the antibody, and incorporated into a suitable vector, which is then introduced into a host to produce the antibody. The present invention encompasses such recombinant antibodies as well.

The antibody of the present invention may be an antibody fragment or a modified antibody, so long as it binds to a protein of the present invention. For example, antibody fragments include Fab, F(ab')₂, Fv, or single chain Fv in which the H chain Fv and the L chain Fv are suitably linked via a linker (scFv, Huston, J.S. et al., Proc. Natl. Acad. Sci. USA, (1988) 85: 5879-5883) . Specifically, antibody fragments can be produced by treating an antibody with an enzyme, for example, papain, pepsin, etc. Alternatively, a gene encoding any of the antibody fragments can be constructed, introduced into an expression vector, and then expressed in suitable host cells (for example, see Co, M. S. et al., J. Immunol., (1994) 152: 2968-2976; Better, M. and Horwitz, A. H., Methods Enzymol., (1989) 178: 476-496; Pluckthun, A. and Skerra, A., Methods Enzymol., (1989) 178: 497-515; Lamoyi, E., Methods Enzymol., (1986) 121: 652-663; Rousseaux, J. et al., Methods Enzymol., (1986) 121: 663-669; Bird, R. E. and Walker, B. W., Trends Biotechnol., (1991) 9: 132-137).

Any antibodies bound to various molecules, such as polyethylene glycol (PEG), can be used as modified antibodies. The "antibody" in the context of the present invention encompasses such modified antibodies as well. To obtain such a modified antibody, the antibody obtained may be chemically modified. These methods are well established in the art.

The antibody of the present invention may be obtained as a chimeric antibody, comprising a variable region derived from a non-human antibody and a constant region derived from a human antibody by using conventional techniques. Alternatively, the antibody of the present invention may be obtained as a humanized antibody, comprising a complementarity determining region (CDR) derived from a non-human antibody, a framework region (FR) derived from a human antibody, and a constant region.

Antibodies thus obtained can be purified to a homogenous state. The antibodies used in the present invention may be separated and purified by any conventional methods used for separation and purification of proteins. There is no limitation to such method at all. Concentration of the above mentioned antibodies can be determined by measuring absorbance, or by the enzyme-linked immunosorbent assay (ELISA), etc.

Assays for antigen-binding activity of the antibody of the present invention include, but are not limited to, ELISA, enzyme immunoassay (EIA), radio immunoassay (RIA), and immunofluorescence. For example, when ELISA is used, a protein of the present invention is placed in a plate coated with the antibody of the present invention, and subsequently, a sample containing the antibody of interest, for example, a culture supernatant of the cells producing the antibody or a purified antibody, is added to the plate. A secondary antibody that recognizes the antibody, labeled with an enzyme such as alkaline phosphatase, is added to the plate, which is then incubated and washed. Subsequently, an enzyme substrate, such as p-nitrophenyl phosphate, is added to the plate, and the antigen-binding activity is estimated by measuring the absorbance. As a protein, a fragment of the protein, such as a fragment comprising the C-terminal or N-terminal region, may be used. To evaluate the activity of the antibody of the present invention, BIAcore (Pharmacia) may be used.

By using these technique, a method for detecting or determining the proteins of the present invention can be carried out, which method comprises the steps of contacting an antibody of the present invention with a sample presumed to contain a protein of the present invention and of detecting or determining the immune complex formed between the antibody and the protein. Since the method of the present invention for detecting or determining proteins can specifically detect or assay the proteins, it is useful in various experiments using proteins.

In addition, the present invention also provides nucleotides specifically hybridizing to the DNA of the nucleotide sequences shown in SEQ ID NOs : 1 , 3 , 5, 7 and 9, (or complementary DNA thereof), which nucleotides have a chain length of at least 15 nucleotides. As used herein, the term "specifically hybridizing" indicates that cross-hybridization does not significantly occur with DNA encoding other proteins under the usual hybridization conditions, preferably under stringent hybridization conditions. Such nucleotides are available as probes for detecting or isolating DNA that encodes a protein of the present invention, or as a primer for amplification. Taking the temperature for hybridization reaction, duration of the reaction, concentration of the probe or primer, length of the probe or primer, ionic strength, and others into account, those skilled in the art can properly select the stringency for the specific hybridization.

The mouse "Tespec PRO-1" and "Tespec PRO-2" genes of the present invention are specifically expresses in the testis. It is also believed that the genes are specifically expressed in mouse germ cells of 18 day old or older. Accordingly, these DNA can also be available as markers (diagnostics) for germ cells. In addition, since the genes of the present invention are thought to be involved in sperm differentiation and maturation, and/or sperm functions including the establishment of fertilization, these DNA are available for examination of infertility.

Further, "nucleotides specifically hybridizing to DNA comprising any one of the nucleotide sequences shown in SEQ ID NOs : 1, 3, 5, 7 and 9 (or complementary DNA thereof), which nucleotides have a chain length of at least 15 nucleotides" also include, for example, antisense oligonucleotides and ribozymes. An antisense oligonucleotide acts on a cell that produces a protein of the present invention to bind to DNA or mRNA encoding the protein, thereby inhibiting the transcription or translation, or enhancing degradation of the mRNA. Antisense oligonucleotides thus inhibit the expression of the proteins of the present invention, resulting in suppression of the functions of the proteins of the present invention. Such antisense oligonucleotides include, for example, an antisense oligonucleotide capable of hybridizing to a definite region of the nucleotide sequences shown in SEQ ID NOs: 1, 3, 5, 7 and 9. Such antisense oligonucleotides are preferably antisense oligonucleotides complementary to at least consecutive 15 nucleotides contained in any of the nucleotide sequences shown in SEQ ID NOs : 1, 3, 5, 7 and 9. More preferably, the above- mentioned antisense oligonucleotides have at least 15 continuous nucleotides containing the translation start codon.

Derivatives or modifications of the antisense oligonucleotides can also be used as antisense oligonucleotides. Such modifications include, but are not limited to, for example, lower alkyl phosphonate modifications, such as methyl-phosphonate or ethyl-phosphonate types; phosphorothioate modifications or phosphoroamidate-modifications, etc.

The antisense oligonucleotides include not only those having the nucleotides complementary to all the corresponding sequence of those constituting the given region of the DNA or mRNA, but also the oligonucleotides having one or more mismatches, as long as the DNA or mRNA and the oligonucleotides can selectively and stably hybridize with any of the nucleotide sequences of SEQ ID NOs: 1, 3, 5, 7 and 9. Such oligonucleotides are nucleotide sequence regions comprising at least 15 continuous nucleotides and exhibiting at least 70% homology, preferably at least 80% homology, more preferably at least 90% homology, most preferably at least 95% homology to the nucleotide sequence. The algorithm to determine the sequence homology mentioned in the references above.

The antisense oligonucleotides of the present invention can be made into an external:preparation, such as a liniment or poultice, by mixing with a suitable base material which is inactive against the antisense oligonucleotides. Also, as needed, the antisense oligonucleotides can be formulated into tablets, powders, granules, capsules, liposome capsules, injections, solutions, nose-drops, and freeze-dried agents by adding excipients, isotonic agents, solubilizers, stabilizers, preservatives, pain-killers, etc. These can be prepared using the usual methods.

The antisense oligonucleotide derivatives of the present invention can be applied both in vivo and in vitro. They can be administered to the patient by directly applying onto the ailing site, or by injecting into a blood vessel and such, so that it will reach the ailing site. An antisense-mounting material can also be used to increase durability and membrane-permeability. Such materials include, but are not limited to, for example, liposome, poly-L lysine, lipid, cholesterol, lipofectin, and derivatives of these.

The dosage of the antisense oligonucleotide derivative of the present invention can be adjusted suitably according to the patient's condition and used in desired amounts. For example, a dose ranging from 0.1 to 100 mg/kg, preferably 0.1 to 50 mg/kg, can be administered.

### Brief Description of the Drawings

Figure 1 shows the mouse "Tespec PRO-1" cDNA sequence and the amino acid sequence thereof. The active sites of trypsin-family serine protease are indicated by underlines. The poly A signal is marked with a wavy line.
Figure 2 shows mouse "Tespec PRO-2" cDNA sequence and the amino acid sequence thereof. The active sites of trypsin-family serine protease are indicated by underlines. The poly A signal is marked with a wavy line.
Figure 3 shows an alignment of amino acid sequences of mouse "Tespec PRO-1", "Tespec PRO-2" and known proteases. Amino acids conserved among all the proteins are marked with "*" and amino acids with similar characteristics are marked with " · ". The active sites of trypsin-family serine protease are boxed.
Figure 4 shows a result of amplification of the cDNA for mouse "Tespec PRO-1" and "Tespec PRO-2" by RT-PCR using mouse testis RNA. Positions of primers used are indicated in the top panel and the electrophoretic pattern of the products amplified by RT-PCR is indicated in the bottom panel.
Figure 5 shows a schematic illustration indicating the structures of mouse "Tespec PRO-1" and "Tespec PRO-2" as well as splicing isoforms thereof. The numbers indicated below the boxes are the numbers of the nucleotides.
Figure 6 shows tissue-specific expression of mouse "Tespec PRO-1" and "Tespec PRO-2" by RT-PCR. Positions of the primers used are indicated in the top panel and the electrophoretic pattern of the products amplified by RT-PCR is indicated in the bottom panel. 1; liver, 2; brain, 3 ; thymus, 4; heart, 5 ; lung, 6; spleen, 7; testis, 8; ovary, 9; kidney, 10; fetus of day 10-11, 11; distilled water (control).
Figure 7 shows tissue-specific expression of mouse "Tespec PRO-1" and "Tespec PRO-2" investigated by Northern blotting. Positions of the primers used are indicated in the top panel and the result of the Northern blotting is indicated in the bottom panel. 1; 7-day-old embryo, 2; 11-day-old embryo, 3; 15-day-old embryo, 4; 17-day-old embryo, 5; heart, 6; brain, 7; spleen, 8; lung, 9; liver, 10; skeletal muscle, 11; kidney, 12; testis.
Figure 8 shows the time of expression of mouse "Tespec PRO-1" and "Tespec PRO-2" in the testis by RT-PCR analysis. 1; W/Wv testis No.1, 2; W/Wv testis No.2, 3; W/Wv testis No.3, 4; testis of 4 days after birth, 5; testis of 8 days after birth, 6; testis of 12 days after birth, 7; testis of 18 days after birth, 8; testis of 42 days after birth, 9; adult testis, 10; adult liver, 11; distilled water (control).
Figure 9 shows the human "Tespec PRO-2" cDNA sequence and the amino acid sequence thereof. The active sites of trypsin-family serine protease are indicated by underlines. The poly A signal is marked with a wavy line.
Figure 10 shows a comparison of nucleotide sequence between mouse and human "Tespec PRO-2". The nucleotides conserved between the two are boxed.
Figure 11 shows a comparison of amino acid sequence between mouse and human "Tespec PRO-2". Amino acid residues shared between the two are indicated by "*" and amino acid residues with similar characteristics are indicated by "·". The active sites of trypsin-family serine protease are boxed.
Figure 12 shows a result of PCR for chromosomal mapping of human "Tespec PRO-2".
Figure 13 shows the nucleotide and amino acid sequences of human "Tespec PRO-3" cDNA. The active sites of trypsin-family serine protease are indicated by underlines. The poly A signal is marked with a wavy line.
Figure 14 shows a comparison of nucleotide sequence homology in regard to "Tespec PRO-1" and "Tespec PRO-3". Homologies of the nucleotide sequences are compared using full-length of mouse Tespec PRO-1", an about 400-bp region of EST from mouse "Tespec PRO-3", and an about 200-bp region of human "Tespec PRO-3" obtained by RT-PCR under a low stringency condition as described in Example 9.
Figure 15 shows the mouse "Tespec PRO-3" cDNA sequence and the amino acid sequence thereof. The active sites of trypsin-family serine protease are indicated by underlines. The poly A signal is marked with a wavy line.
Figure 16 shows a comparison of nucleotide sequence between mouse "Tespec PRO-3" (m. Tespec PRO-3) and human "Tespec PRO-3" (h. Tespec PRO-3). Nucleotides conserved between the two are boxed.
Figure 17 shows a comparison of amino acid sequence between mouse "Tespec PRO-3" (m. Tespec PRO-3) and human "Tespec PRO-3" (h. Tespec PRO-3). Amino acid residues conserved between the two are boxed.

### Best Mode for Carrying out the Invention

The present invention is illustrated more specifically below with reference to Examples, but is not to be construed as being limited to the examples described below.

### Example 1. Isolation of "Tespec PRO-1" gene fragment

A mixture of plasmids derived from 5 x 10⁴ clones was isolated and purified from a plasmid library of mouse heart cDNA (GIBCO, 5 x 10⁹ cfu/ml). By using the plasmid mixture as a template, PCR amplification was performed according to the following procedure, using the primer "76A5sc2-B" specific to the gene that was named "76A5sc2" by the present inventors and the vector primer "SPORT RV".

SuperScript Mouse heart cDNA library and SuperScript Mouse testis cDNA library (GIBCO, 5 x 10⁹ cfu/ml) were diluted 1:100. µl aliquots of the diluted solutions were added to each of 16 tubes containing 3 ml of LB-Amp medium, and the mixtures were incubated at 30 °C. Then the mixtures of plasmids were prepared with the QIAspin mini-prep kit (QIAGEN) (each plasmid preparation contains mixture of plasmids derived from 5 x 10⁴ independent clones). Using the plasmids from the mouse heart cDNA library as templates, PCR was carried out with Ampli Taq Gold (Perkin Elmer) as polymerase and the primer pair of 76A5sc2-B (SEQ ID NO: 11 / 5'-GAT CMA CAG GTG CCA GTC ATC A-3') and SPORT SP6 (SEQ ID NO: 12/ 5'-ATT TAG GTG ACA CTA TAG AA-3'). The thermal cycling profile was: a pre-heat at 95°C for 12 minutes, 40 cycles of denaturation at 96°C for 20 seconds, annealing at 55°C for 20 seconds and extension at 72°C for 2 minutes, and subsequent final extension at 72°C for 3 minutes.

The PCR reactions were subjected to electrophoresis on a 1.5% agarose gel. PCR products of about 0.7 Kb were cut out from the gel and then recovered by QIAquick Gel Extraction Kit (QIAGEN). The PCR products were cloned into pGEM T easy vectors (PROMEGA) by TA cloning using T4 DNA ligase (PROMEGA).

Eight colonies were selected from the colonies emerged, and the inserted fragments were amplified by colony PCR as follows.

The bacteria from each colony, which contain the recombinant gene, were directly suspended in 20 µl of PCR reaction solution containing a pair of the primers, SPORT FW (SEQ ID NO: 13/ 5'-TGT AAA ACG ACG GCC AGT-3') and SPORT RV (SEQ ID NO: 14/ 5'-CAG GAA ACA GCT ATG ACC-3'), and KOD dash polymerase. PCR was performed by employing a thermal cycling profile of pre-heat at 94 °C for one minute, subsequently 32 cycles of denaturation at 96°C for 15 seconds, annealing at 55°C for 5 seconds, and extension at 72°C for 25 seconds..

The amplification of the PCR products of interest was verified by agarose gel electrophoresis. If desired, the PCR products were purified by gel filtration with Microspin S-300 or S-400 (Pharmacia).

The PCR products from the above colony PCR or RT-PCR, were used as templates for sequencing. After the PCR reaction, the products generated were examined by agarose gel electrophoresis. If the products were contaminated, the PCR product of interest was cut out from the agarose gel to remove the contaminants. Otherwise, the products were purified by the above-mentioned gel filtration. Sequencing was performed by cycle sequence using Dye Terminator Cycle Sequencing FS Ready Reaction Kit, dRhodamine Terminator Cycle Sequencing FS ready Reaction Kit, or BigDye Terminator Cycle Sequencing FS ready Reaction Kit (Perkin-Elmer). Primers used were SPORT FW and SPORT RV. Unreacted primers, nucleotide monomers, and the like were removed by using a 96-well precipitation HL kit (AGTC). The nucleotides sequences were determined in the ABI 377 or ABI 377XL DNA Sequencer (Perkin-Elmer).

The result showed that seven plasmids contained the nucleotide sequence of 76A5sc2 and a single plasmid contained a distinct nucleotide sequence (the size of insert was about 0.5 Kb). This nucleotide sequence was then analyzed by searching the GCG database. Since this nucleotide sequence had an ORF, it was translated into an amino acid sequence. The amino acid sequence was also analyzed by searching the GCG database. The results showed that this gene fragment contained regions homologous to a number of known trypsin-family serine proteases at the nucleotide and amino acid levels. However, no known genes showed significant homology to this gene fragment over the entire regions, suggesting that this gene fragment has a novel origin. Further, the amino acid sequence was revealed to have a "Trypsin-His (PROSITE PS00134)" motif, one of the trypsin-family serine protease motifs. This also suggests that the gene fragment is derived from a novel protease gene.

### Example 2. Cloning of full-length cDNA of the "Tespec PRO-1" gene

By using the plasmid obtained from the SuperScript Mouse heart cDNA library in Example 1 as a template, plasmid library RACE was carried out employing Ampli Taq Gold as polymerase. The primer sets used in this experiment were a pair of No9-C (SEQ ID NO: 15/ 5'-ATG CTT CTG CTA TCG TGG AAG G-3') which was newly designed based on the gene fragment isolated in Example 1, and a vector primer, SPORT FW or SPORT T7 (SEQ ID NO: 16/ 5'-TAA TAC GAC TCA CTA TAG GG-3'), and a pair of the primer No9-B (SEQ ID NO: 17/ 5'-CTT TGT GCT GAG GTC TTC AGT G-3'), which was newly designed based on the gene fragment and a vector primer, SPORT RV. The thermal cycling profile of the PCR was: a pre-heat at 95°C for 12 minutes, 42 cycles of denaturation at 96°C for 20 seconds, annealing at 55°C for 20 seconds and extension at 72°C for 5 minutes, and subsequent final extension at 72°C for 3 minutes.

The PCR products were identified by agarose gel electrophoresis. Further, for these PCR products, the nucleotide sequences were determined directly or after cloned into pGEM T easy vector.

Since two PCR bands were obtained by 3' RACE, the nucleotide sequences thereof were determined. The sequencing revealed that one of the two had the nucleotide sequence of the other in which a poly A stretch is attached to an internal site in the nucleotide sequence.

Likewise, 5' RACE also gave two PCR bands with different sizes. DNAs from the respective bands were subcloned, and their nucleotide sequences were determined. The result revealed that the two were identical to each other in nucleotide sequence at the 3' end, indicating that the two were different isoforms produced by alternative splicing.

The nucleotide sequences from the shorter band generated by 5' RACE and the longer band generated by 3' RACE were ligated to each other to give a nucleotide.sequence encoding the entire protease, which was designated "Tespec PRO-1" (Testis specific expressed serine proteinase-1).

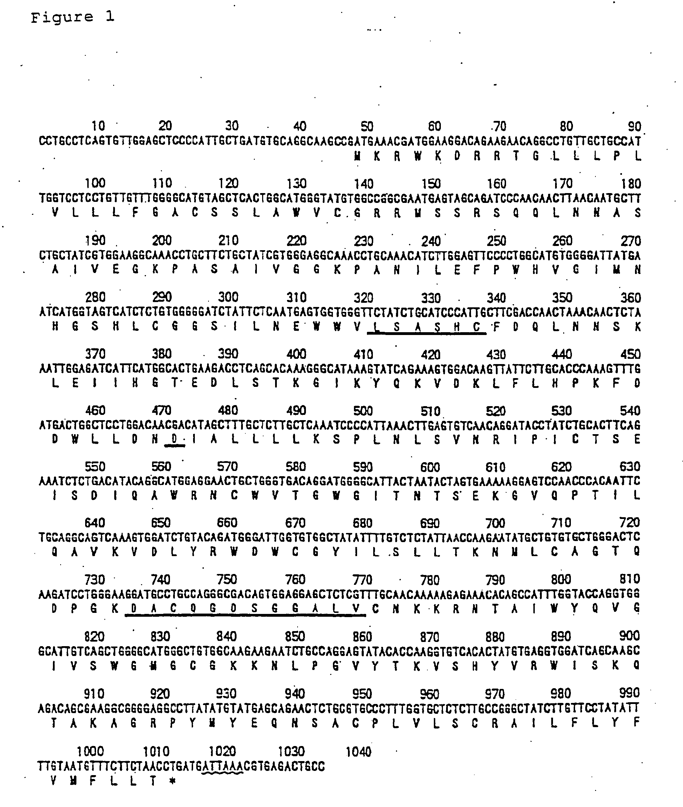

The resulting "Tespec PRO-1" cDNA contains 1033 nucleotides and is predicted to code for 321 amino acids (Figure 1). The nucleotide sequence is shown in SEQ ID NO: 1 and the amino acid sequence is illustrated in SEQ ID NO: 2. The amino acid sequence contains a hydrophobic region at its N terminus, which is predicted to be a signal peptide. The amino acid sequence also has a region rich in hydrophobic amino acids at its C-terminus.

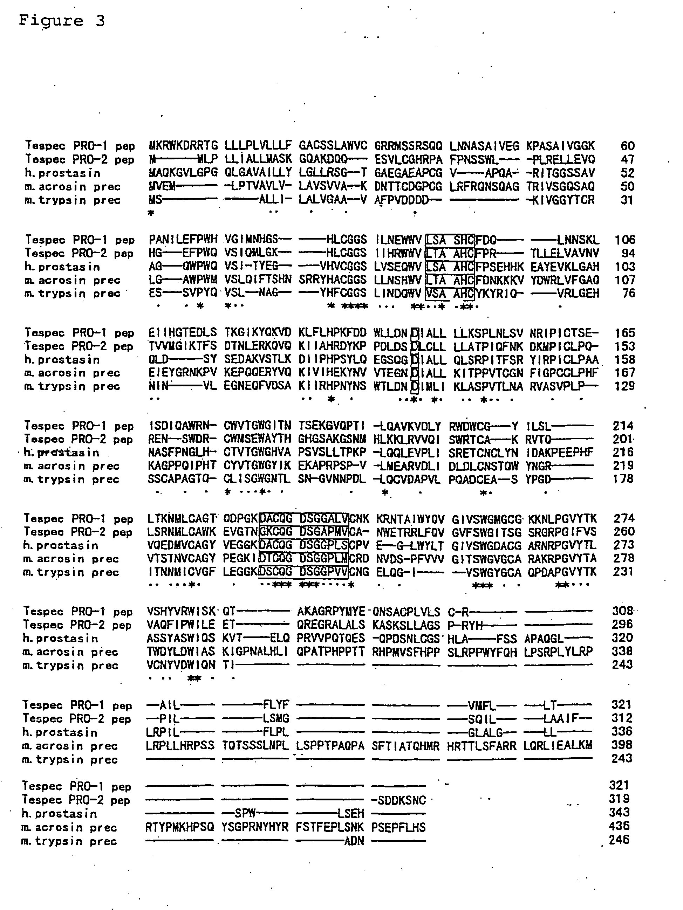

Based on the analytical search of the GCG, the amino acid sequence was proved to contain two types of trypsin-family serine protease motifs, "Trypsin-His (PROSITE PS00134)" and "Trypsin-Ser (PROSITE PS00135)". PROSITE indicates "if a protein includes both the serine and histidine active site signatures, the probability of it being a trypsin family serine protease is 100%" (Brenner, S., 1988, Nature, 334: 528-530; Rawlings, N. D. and Barrett, A. J. (1994) Meth. Enzymol., 244: 19-61). "Tespec PRO-1" therefore can be regarded as a trypsin-family serine protease. The nucleotide sequence of this gene and its deduced amino acid sequence were analyzed by searching the GCG database. The results showed that the two motifs mentioned above and flanking region thereof exhibits high homologies to known trypsin-family serine proteases, such as acrosin, prostasin and trypsin. It was also revealed that the positions of aspartic acid residues required for the protease activity and the cysteine residues anticipated to be responsible for intramolecular disulfide bonding are well conserved relative to other proteases (Figure 3). For the other region, however, no known genes or proteins were found to exhibit significant homology to this sequence at the nucleotide and amino acid levels, revealing that this protein is a novel trypsin-family serine protease.

### Example 3. Cloning of full-length cDNA of the "Tespec PRO-2" gene

For the band with larger molecular weight (the band with a nucleotide sequence different from that of "Tespec PRO-1" at the 5' end), which was obtained during the cloning of "Tespec PRO-1" by 5' RACE in Example 2, 3' and 5' RACE were carried out using newly synthesized primers designed based on the nucleotide sequence of "Tespec PRO-1" (No9-G or No9-J) as well as using, as templates, the plasmid mixture obtained from the SuperScript Mouse testis cDNA library in Example 1.

Specifically, PCR was conducted by using primer pairs of No9-G (SEQ ID NO: 18/ 5'-CAG TCA ATG TCA CTG TGG TCA T-3') and SPORT FW, and No9-J (SEQ ID NO: 19/ 5'-ACT TGC CGT TGG TGC CCA CTT C-3') and SPORT RV. In this PCR, Ampli Taq Gold was used as polymerase and its thermal cycling profile was as follows: a pre-heat at 95°C for 12 minutes, 42 cycles of denaturation at 96°C for 20 seconds, annealing at 55°C for 20 seconds and extension at 72°C for 5 minutes, and subsequent final extension at 72°C for 3 minutes.

The nucleotide sequences of the PCR products were determined directly or after cloned into pGEM T easy vector.

Two 3' RACE products were obtained by 3' RACE, both of which were sequenced. By this analysis, the two nucleotide sequences were showed to have an identical region at their 5' ends but distinct regions at their 3' ends. One of the sequences was identical to the aforementioned nucleotide sequence having the sequence of "Tespec Pyro-1" in which a poly A stretch is attached to an internal site of the sequence. The other sequence contained a nucleotide sequence different from that of "Tespec PRO-1" at its 3' end.
Multiple bands were given by 5' RACE. Those bands were subcloned, and their nucleotide sequences were determined. The result showed that all these bands shares an identical 3' terminal sequence. Thus they are shown to be splicing isoforms. Since one of the 5' RACE products has a long ORF, the 5' RACE product and the above-mentioned 3'RACE product whose nucleotide sequence is different from that of "Tespec PRO-1" at the 3' end were assembled together, thereby giving a nucleotide sequence presumed to encode a protease. This sequence was named "Tespec PRO-2". The nucleotide sequence is shown in SEQ ID NO: 3, and the deduced amino acid sequence is indicated in SEQ ID NO: 4.

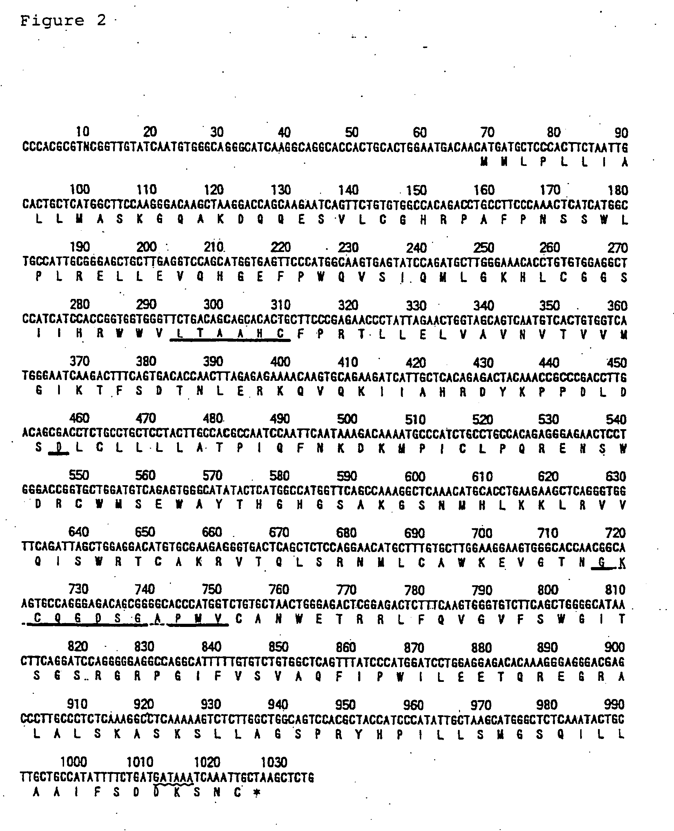

"Tespec PRO-2" cDNA thus obtained consists of 1034 nucleotides (Figure 2) and its 5' non-coding region consists of 68 nucleotides. By contrast, the 3'-non-coding region of this cDNA is very shorter, consisting of only nine nucleotides. A putative poly A signal found in this cDNA is GATAAA, and it is predicted to be weaker signal as compared to the signal generally recognized in mRNAs (AAUAAA) . Based on the sequence of this cDNA, "Tespec PRO-2" is predicted to encode 319 amino acids, which contains a possible region of signal peptide at its N-terminus. But, unlike "Tespec PRO-1", the protein does not contain a region rich in hydrophobic amino acids at its C-terminus. While the amino acid sequence contains a trypsin-family serine protease motif, "Trypsin-His", the "Trypsin-Ser" motif of this protein (GKCQGDSGAPMV) contains 2 amino acid residues that are deviated from the consensus sequence of the motif that consists of 12 amino acid residues ([DNSTAGC] - [GSTAPIMVQH]-X-X-G-[DE]-S-G-[GS] - [SAPHV]-[LIVMFYWH] - [LIVMFYSTANQH]). However, some known trypsin-family serine proteases have sequences that are different from the consensus sequence at several amino acid residues. "Tespec PRO-2" obtained is predicted to function as a protease.

The nucleotide sequence of "Tespec PRO-2" and its deduced amino acid sequence were analyzed by searching the GCG database. The results showed that, like "Tespec PRO-1", the two motifs of "Tespec PRO-2" mentioned above and flanking region thereof exhibits high homologies to known trypsin-family serine proteases. It was also revealed that the positions of aspartic acid residues required for the protease activity and the cysteine residues anticipated to be responsible for intramolecular disulfide bonding are highly conserved relative to other proteases (Figure 3). For the other region, however, no known genes or proteins were found to exhibit significant homology at the nucleotide and amino acid levels, revealing that this protein is a novel trypsin-family serine protease.

### Example 4. Splicing isoforms of "Tespec PRO-1" and "Tespec PRO-2"

Homologies between "Tespec PRO-1" and "Tespec PRO-2" were 52.2% and 33.1% at the nucleotide and amino acid levels, respectively. These values are of similar extent, compared to those of other known trypsin-family serine proteases.

The splicing isoform of "Tespec PRO-2" obtained by 5' RACE in Example 3 does not appear to encode a protease, since it contains multiple termination codons in the nucleotide sequence at the splicing junction and in the region that is missing in "Tespec PRO-2", which will prevent ORF extending. The splicing isoform was analyzed in more detail by RT-PCR as follows.

Based on the nucleotide sequence obtained by cDNA cloning, primers were synthesized which include No9-P (SEQ ID NO: 20/ 5'-GCA CTG GAA TGA CAA CAT GAT GC-3'), No9-Q (SEQ ID NO: 21/ 5'- ATT GTC GTG .._ GCA AGT AGG AGC A-3'), No9-N (SEQ ID NO: 22/ 5'-CGA GTC TCC CAG TTA GCA CAG A-3'), No9-M' (SEQ ID NO: 23/ 5'-CGG TGA CTT GGT CAT GTC TGT G-3'), No9-K (SEQ ID NO: 24/ 5'-GGA TCC ATG AAA CGA TGG AAG GAC AGA AG-3'), No9-G, No9-J, and No9-O (SEQ ID NO: 25/ 5'-CGC AGA GTT CTG CTC ATA CAT A-3'). RT-PCR was performed by using these primers, cDNAs prepared from mouse tissue as templates, Ampli Taq Gold as polymerase and the thermal cycling profile of: pre-heating at 95°C for 12 minutes, 40 cycles of denaturation at 96°C for 20 seconds, annealing at 60°C for 20 seconds and extension at 72°C for 1 minute, and subsequent final extension at 72°C for 3 minutes. PCR reactions were subjected to electrophoresis on a 1.5% Seakem GTG agarose (TaKaRa).

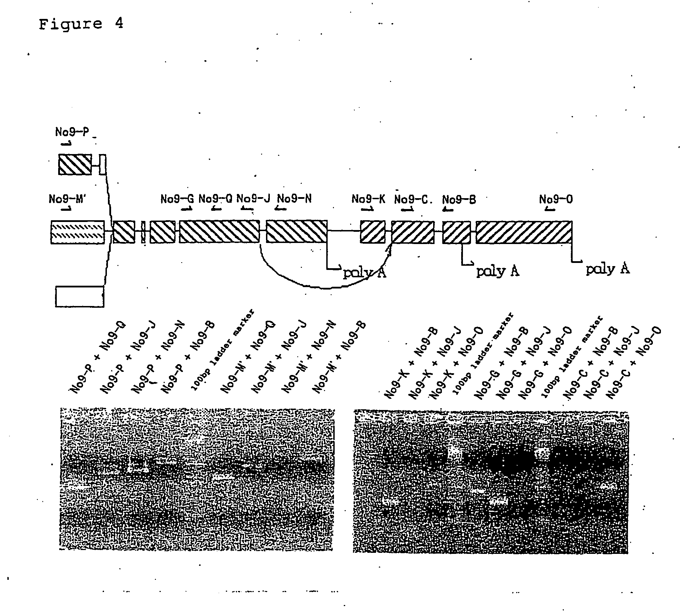

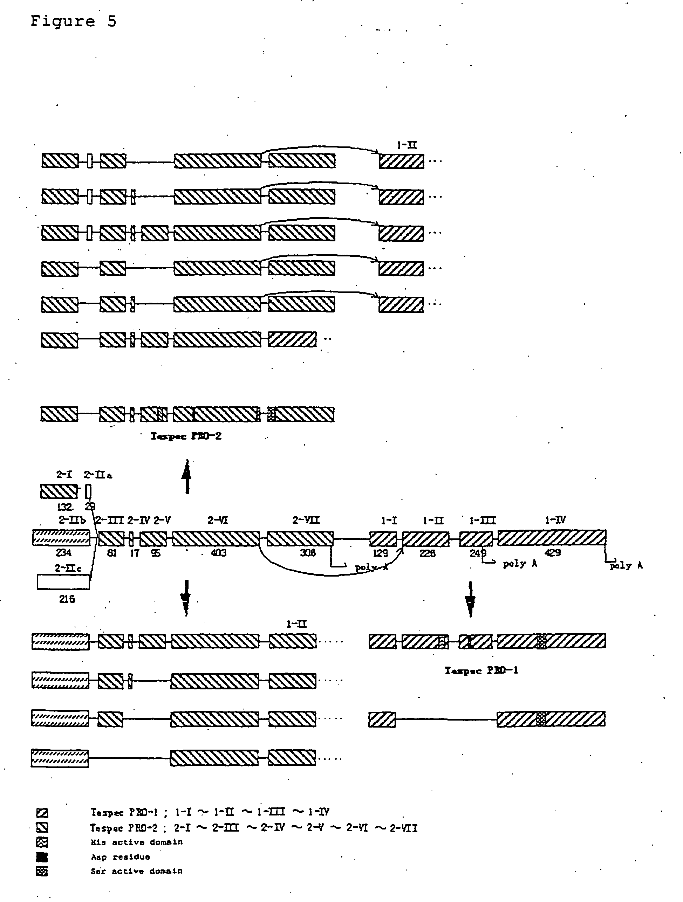

The results of RT-PCR analysis (Figures 4 and 5) showed that isoforms having the boxes (2-I)-(2-III)-(2-VI) at the 5' end were appear to be dominant in the population of the splicing isoforms of "Tespec PRO-2". The population appears to be larger than that of "Tespec PRO-2". The RT-PCR analysis has verified cDNA isoforms with Box 2-1 in which the Box is connected via Box 2-VI to Box 2-VII or Box 1-II (the latter is suspected to be a chimeric cDNA molecule with "Tespec PRO-1") . In contrast, the analysis also revealed that there is only a single type of cDNA isoform with Box 2-IIb, a chimeric cDNA with "Tespec PRO-1" in which the Box is connected via Box 2-VI to Box 1-II (Figures 4 and 5). Such chimeras may be formed because "Tespec PRO-2" and "Tespec PRO-1" are located in the close proximity on the chromosome, as well as due to weak signal intensity of the poly A signal in "Tespec PRO-2". It remains to be clarified why such splicing isoforms (encoding only short proteins) that are seemingly meaningless exist. However, there is a possibility that the expression of "Tespec PRO-2" is regulated by splicing as well as transcriptionally.

### Example 5. Tissue distribution of the "Tespec PRO-1" and "Tespec PRO-2" genes

Tissue distribution of "Tespec PRO-1" and "Tespec PRO-2" were investigated by RT-PCR. Total RNAs (Ambion) isolated from 10 types of adult mouse tissue (liver, brain, thymus, heart, lung, spleen, testis, uterus, kidney, and fetus of day 10-11) were ..used to synthesize cDNA by reverse transcription using SuperScript II (GIBCO) as a reverse transcriptase and using (dT)₃₀ VN primer. The resulting cDNAs were used as templates for RT-PCR. QUICK-Clone cDNA from mouse 7-day embryo as well as 17-day embryo (CLONTECH) was also used as a template for RT-PCR.

"Tespec PRO-1"-specific primers used were No9-A (SEQ ID NO: 26/ 5'-GGC ATG TAG CTC ACT GGC ATG-3') and No9-B. "Tespec PRO-2"-specific primers used were 29(-) (SEQ ID NO: 27/ 5'-GGA CCA GCA AGA ATC AGT TCT G-3') and 17(+)95(+) (SEQ ID NO: 28/ 5'-CTG CTA CCA GTT CTA ATT TGC C-3'). G3PDH control primers used were G3PDH 5' (SEQ ID NO: 29/ 5'-GAG ATT GTT GCC ATC AAC GAC C-3') and G3PDH 3' (SEQ ID NO: 30/ 5'-GTT GAA GTC GCA GGA GAC AAC C-3'). Polymerase used was Ampli Taq Gold and the thermal cycling profile of PCR was: pre-heat at 95°C for 12 minutes, 42 cycles of denaturation at 96°C for 20 seconds, annealing at 60°C for 20 seconds and extension at 72°C for 30 seconds (28 cycles for G3PDH), and subsequent final extension at 72°C for 3 minutes. The PCR reactions were subjected to electrophoresis on a 1.5% Seakem GTG agarose (TaKaRa).

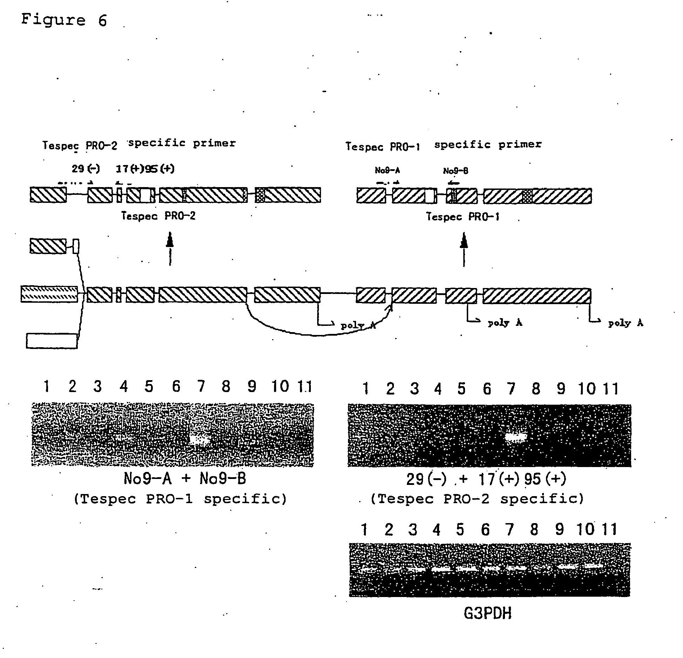

The result showed that both "Tespec PRO-1" and "Tespec PRO-2" were expressed in the testis at high levels (Figure 6). Interestingly, it was also shown that these genes, despite of being cloned from the plasmid library of mouse heart cDNA, were hardly expressed in the heart. In the tissue other than the testis, the bands of interest were observed, though they were very faint.

In addition, tissue distribution was analyzed by mouse MTN blot (CLONTECH), using, as probes, a part of the coding region of "Tespec PRO-1" (the region containing the entire sequence of Box 1-II; the nucleotide positions 110 to 401) and a region in the vicinity of exon 2-VI of "Tespec PRO-2" (nucleotide positions 340 to 723) (this probe may be recognize "Tespec PRO-2" and all the splicing isoforms thereof, since it covers the region that is common to many of the splicing isoforms of "Tespec PRO-2", therefore it is not a "Tespec PRO-2"-specific probe).

The RT-PCR products amplified by using cDNAs from adult mouse testis as templates and No9-A and No9-B primers were labeled with [α-³²p] dCTP by using the Megaprime DNA labeling system (Amersherm)-, and unreacted [α-³²p] dCTP was removed to give the "Tespec PRO-1" probe. Likewise, the "Tespec PRO-2" probe was prepared by PCR using No9-G and No9-J primers and subsequently by labeling with [α-³²P]dCTP. The hybridization was carried out at 68°C by using Mouse Multiple Tissue Northern (MTN) blot and Mouse Embryo Multiple Tissue Northern (MTN) blot (CLONTECH) in ExpressHyb Hybridization Solution (CLONTECH), according to the manufacturer's instruction.

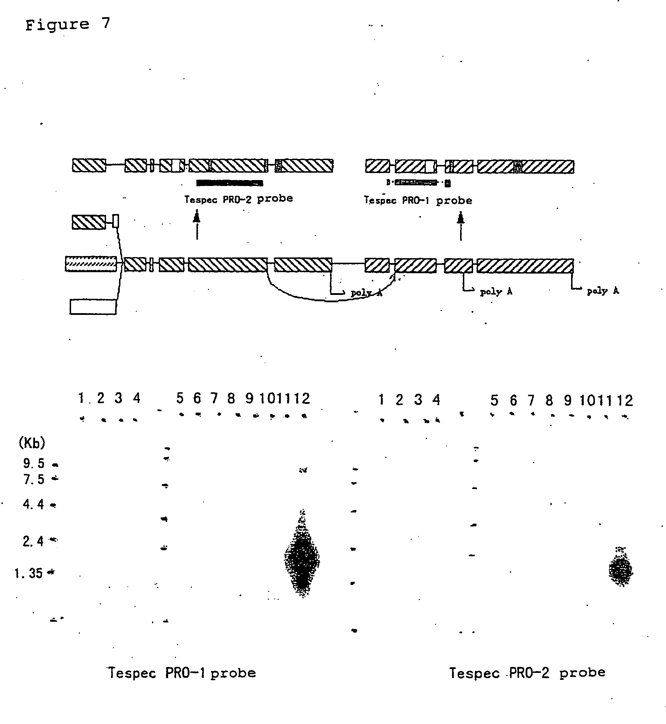

A band about 1.2 Kb in length was observed only in the testis by using the "Tespec PRO-1" probe (Figure 7). This band was not detected in the tissue other than the testis, as well as in the fetus. Like the "Tespec PRO-1" probe, the "Tespec PRO-2" probe also detected an about 1.2-Kb band only in the testis (Figure 7). The band was not detected in tissue other than the testis, as well as in the fetus.

The results described above demonstrate that both "Tespec PRO-1" and "Tespec PRO-2" are specifically expressed in the testis.

### Example 6. Expression times of the "Tespec PRO-1" and "Tespec PRO-2" genes in the testis

In mice, the primordial germ cells emerge in the fetus 7 days after fertilization, and they migrate to the genital ridge (11 days after fertilization) and differentiate into precursor cells of spermatogonium (13 days after fertilization). The precursor cells of spermatogonium enter into the arrested state from then on. They become spermatogonia, germ-line stem cells, after birth and then start their self-proliferation and differentiation into sperm. It takes about 34 days for spermatogonia to differentiate via spermatocytes and spermatids into mature sperm (in actuality, since spermatogonia per se have their own differentiation stage, if this stage is included, the period required for maturation is about 42 days in total). Then, testes of postnatal mice are collected per day after birth to verify the expression of "Tespec PRO-1" and "Tespec PRO-2". This reveals at what stage of differentiation the genes are expressed in the sperm, or whether the genes are expressed in nurse cells (e.g. Sertoli's cells and Leydig's cells) in the testis.

On one hand, there exists a mutant mouse W (White spotting) that has a defect in chromosome 5 (Besmer, P. et al. (1993) Dev. Suppl., 125-137). This mutant mouse has a defect in c-kit, which is a receptor tyrosine kinase and expressed in the spermatogonia and spermatocytes. The mutant mouse has a deficiency in germ cells (complete deficiency) or a differentiation insufficiency (partial deficiency) at the stages after spermatogonium, though it has normal nurse cells such as Sertoli's cells and Leydig's cells in the testis. Thus, the expression of "Tespec PRO-1" and "Tespec PRO-2" were verified in the testis of the mutant mice W/Wv.

RT-PCR was performed by using, as templates, cDNAs prepared from total RNAs isolated from mouse testes 4 days, 8 days, 12 days, 18 days, and 42 days after birth, and from testes of three W/Wv mice 56 days after birth. In this RT-PCR experiment, cDNAs from adult mouse testis and liver were also used. Primers used were the "Tespec PRO-1"-specific primer and "Tespec PRO-2"-specific primer described above in Example 5. In the same manner as described in Example 5, 40 cycles (29 cycles for G3PDH) of PCR was conducted.

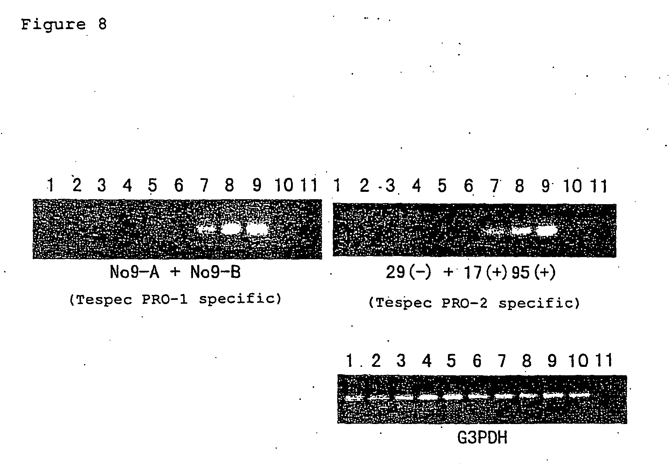

The result of RT-PCR demonstrate that expression levels of "Tespec PRO-1" and "Tespec PRO-2" were elevated in the testis 18 days after birth and later; neither gene was expressed at all before 12 days after birth nor in the testis of W/Wv mutant mouse (Figure 8). No expression of the genes was detected in the liver, a negative control. These results suggest that both "Tespec PRO-1" and "Tespec PRO-2" are expressed not in the nurse cells such as Sertoli's cells and Leydig's cells, but in germ cells, and that their expression levels are elevated in the spermatocytes differentiated from germ cells or in the spermatids after meiosis.

### Example 7. Cloning of full-length cDNA of human "Tespec PRO-2"

Human "Tespec PRO-2" cDNA was cloned, based on the nucleotide sequence of mouse "Tespec PRO-2". Human testis poly A+ RNA (CLONTECH) was converted into cDNA by using the reverse transcriptase SuperScript II (GIBCO) and (dT)₃₀VN primer. PCR was carried out, by using the cDNA as a template as well as using No9-G and No9-Q primers derived from mouse "Tespec PRO-2". Polymerase used was AmpliTaq Gold and the thermal cycling profile of the low stringency PCR was: pre-heat at-95°C for 12 minutes, 42 cycles of denaturation at 96°C for 20 seconds, annealing at 55°C for 20 seconds and extension at 72°C for 30 seconds, and subsequent final extension at 72°C for 3 minutes.

The resulting RT-PCR product was sequenced directly to determine the nucleotide sequence. The result showed that this PCR product is a gene fragment of human "Tespec PRO-2", which exhibits about 80% homology to mouse "Tespec PRO-2" in nucleotide sequence. Based on this nucleotide sequence, primers for 5'RACE, i.e. h-B (SEQ ID NO: 31/ 5'-AGA GGT CAC TGT CGA GCT GAG-3') and h-D (SEQ ID NO: 32/ 5'-TGT GAA TAA TGA CCT TCT GCA C-3'), and primers for 3' RACE, i.e. h-A (SEQ ID NO: 33/ 5'-TTC AGC AAC ATC CAC TCG GAG A-3') and h-C (SEQ ID NO: 34/ 5'-AAG CAA GTG CAG AAG GTC ATT A-3') were generated. Nested 3' and 5' RACE was conduced by using human testis Marathon ready cDNA (CLONTECH) as a template, according to the manufacturer's instruction. As a result, a full-length-cDNA for human "Tespec PRO-2" was cloned successfully. The nucleotide sequence is shown in SEQ ID NO: 5 and the amino acid sequence thereof is shown in SEQ ID NO: 6.

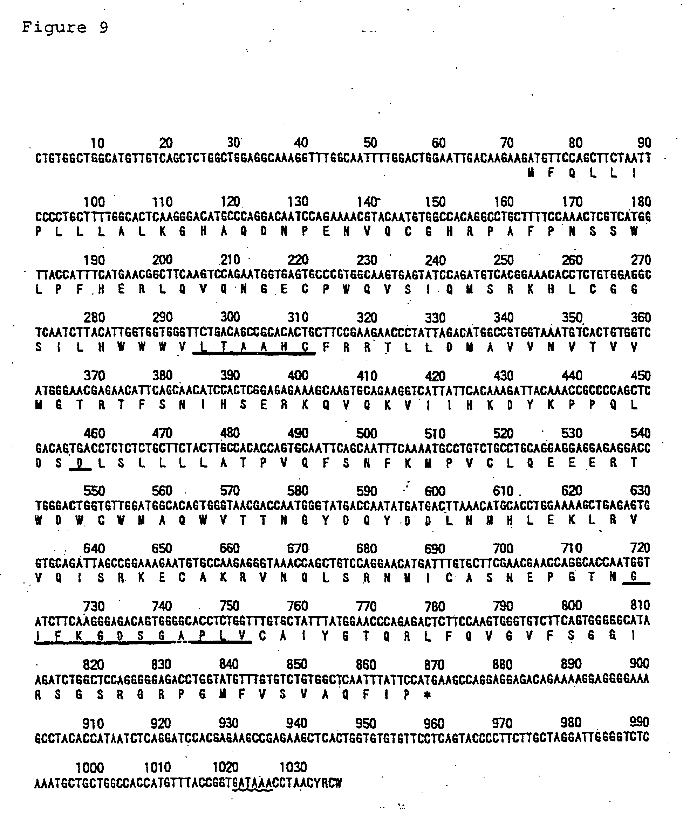

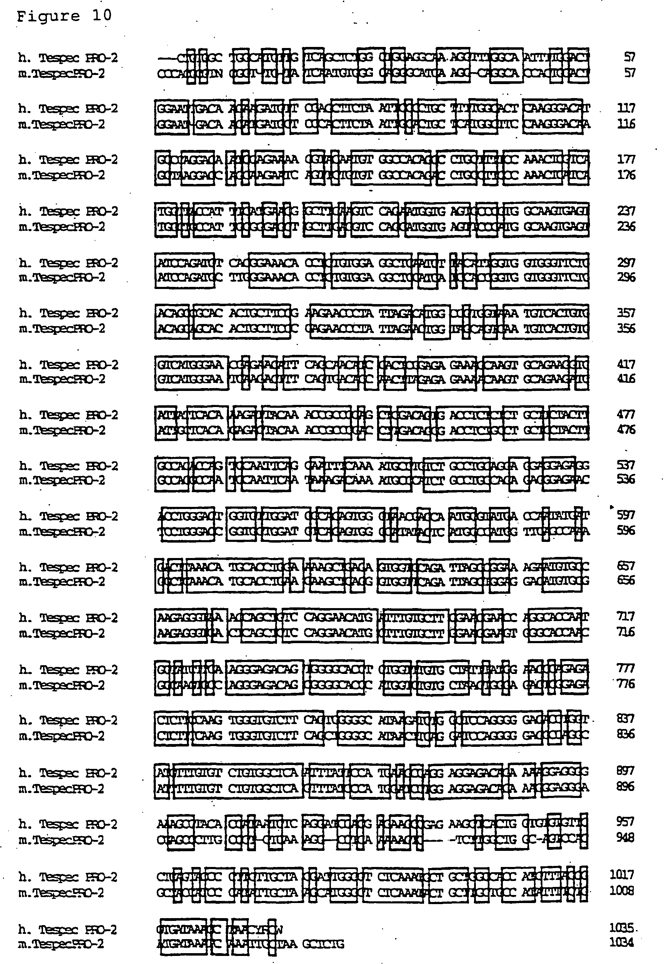

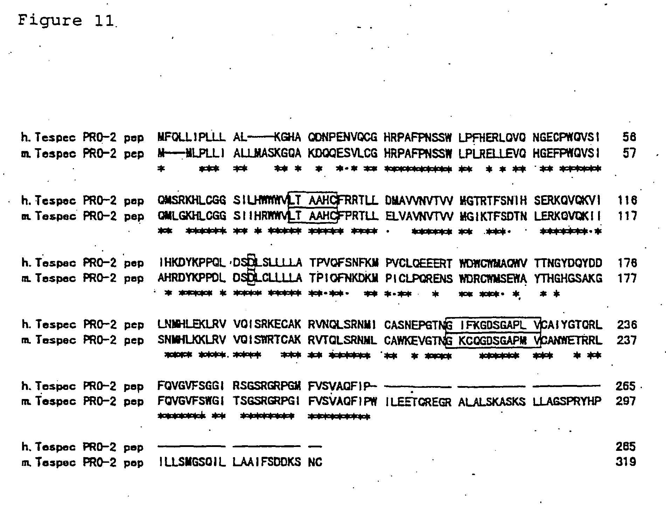

The human "Tespec PRO-2" cDNA consists of 1035 nucleotides and is predicted to encode 265 amino acids (Figure 9). Homology between human and mouse "Tespec PRO-2" is 74.2% at the nucleotide level and 69.8% at the amino-acid level. The amino acid sequence of the human "Tespec PRO-2" is shorter than that of mouse "Tespec PRO-2" by 54 residues at the C-terminus, and consequently, the human nucleotide sequence is longer in the 3'non-coding region as compared with that of the mouse gene (Figure 10 and 11). In addition, there is a region predicted to be a signal peptide at the N-terminus, and the C-terminal region is also rich in hydrophobic amino acids. The deduced amino acid sequence of human "Tespec PRO-2" contains a trypsin-family serine protease motif, "Trypsin-His". The motif of "Trypsin-Ser" of this protein contains an amino acid residue (GIFKGDSGAPLV) that is deviated from the consensus sequence in this motif that consists of 12 amino acid residues ([DNSTAGC]-[GSTAPIMVQH]-X-X-G-[DE]-S-G-[GS]-[SAPHV]-[LIVMFYWH]-[LIVMFYSTANQH]) (mouse "Tespec PRO-2" contains two amino acid residues deviated from the consensus sequence in this motif that consists of 12 amino acid residues).

The result of database search demonstrates that no known genes or proteins exhibit significant homology to the human "Tespec PRO-2", at nucleotide and amino acid levels, revealing that this protein is a novel trypsin-family serine protease.

### Example 8. Chromosomal mapping of human "Tespec PRO-2"

PCR was performed by using a human chromosome panel (CORRIELL CELL REPOSITORIES) as a template, a pair of primers, h-A and h-F (SEQ ID NO: 35/ 5'-CAT TGG TCG TTA CCC ACT GTG C-3), and Advantage cDNA polymerase (CLONTECH) as polymerase. The thermal cycling profile of PCR was: pre-heat at 95°C for 1 minute, 37 cycles of denaturation at 96 °C for 15 seconds, annealing at 60°C for 15 seconds and extension at 68°C for 30 seconds, and subsequent final extension at 68°C for 3 minutes. The PCR reaction was subjected to electrophoresis on a 1.5% Seakem GTG agarose (TaKaRa).

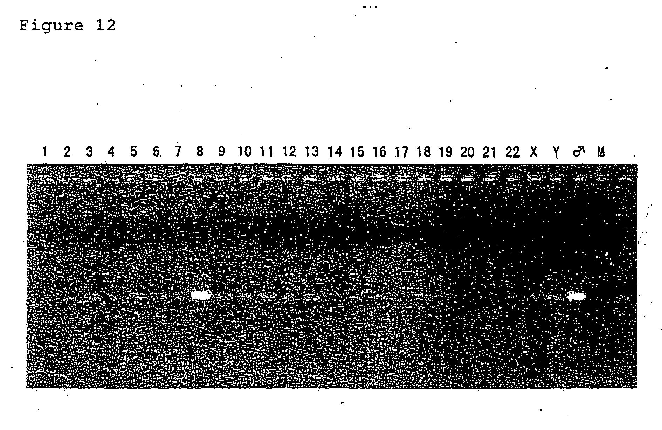

As the result of PCR, human "Tespec PRO-2" was mapped on chromosome 8 (Figure 12).

### Example 9. Cloning of full-lezigth cDNA of the human "Tespec PRO-3" gene

Human testis poly A+ RNA (CLONTECH) was converted into cDNA by using the reverse transcriptase SuperScript II (GIBCO) and (dT)₃₀ VN primer. RT-PCR was carried out by using the cDNA synthesized as a template, and the primer pair of PR01-E (SEQ ID NO: 36/ 5'-ATT CTC AAT GAG TGG TGG GTT CT-3') and PRO1-D (SEQ ID NO: 37/ 5'-CCA GCA CAC AGC ATA TTC TTG G-3') that are synthesized on the basis of the nucleotide sequence of mouse "Tespec PRO-1". The low stringency PCR was performed using the polymerase AmpliTaq Gold and the thermal cycling profile of: pre-heat at 95°C for 12 minutes, 5 cycles of denaturation at 96°C for 20 seconds, annealing at 50°C for 20 seconds, and extension at 72°C for 45 seconds, and subsequent 35 cycles of denaturation at 96°C for 20 seconds, annealing at 60°C for 20 seconds, and extension at 72°C for 45 seconds, and final extension at 72°C for 3 minutes.

The RT-PCR product was purified by gel filtration and then its nucleotide sequence was determined. The sequence analysis has revealed that this product is a gene fragment encoding a trypsin-family serine protease. The translation of this gene fragment revealed that it contained a "Trypsin-His" motif. A database search for the nucleotide sequence of this gene fragment showed that it overlaps in part with the sequence of a human EST (AA781356, aj25c04.s1 Soares-testis-NHT Homo sapiens cDNA clone 1391334 3', mRNA sequence.). Translation of this EST revealed the presence of a "Trypsin-Ser" motif in the amino acid sequence. Then, on the basis of the nucleotide sequence of the gene fragment obtained, primers were prepared: hPR03-B (SEQ ID NO: 38/ 5'-GGA AAC AGC TCC TCG GAA TAT AAG C-3') and hPR03-D (SEQ ID NO: 39/ 5'-TGG ATG GGC TAG TTA AGT CGT TGG T-3') for 5'RACE , and hPR03-A (SEQ ID NO: 40/ 5'-TTC GAG GGA AGA ACT CGG TAT TC-3') and hPR03-C (SEQ ID NO: 41/ 5'-TGT GAA AAC GGA TCT GAT GAA AGC G-3') for 3' RACE. Nested RACE was conducted by using human testis Marathon ready cDNA (CLONTECH) as a template, according to the manufacturer's instruction to clone a full-length cDNA. The product obtained by the RACE was sequenced directly or after subcloned into the pGEM T easy vector. The nucleotide sequence is shown in SEQ ID NO: 9 and the amino acid sequence is shown in SEQ ID NO: 10.

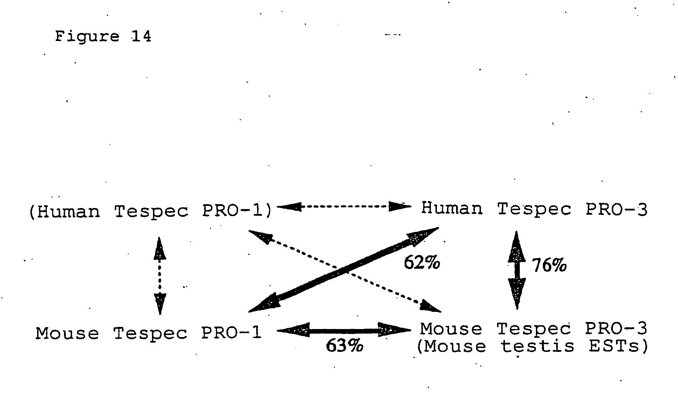

This novel human gene showed higher homology to mouse testis ESTs deposited in the database (AA497965, AA497934, AA497919, etc.) than to mouse "Tespec PRO-1" (Figure 14), though this gene was obtained using the primers generated on the basis of the nucleotide sequence of mouse "Tespec PRO-1". Thus, the gene was designated human "Tespec PRO-3".

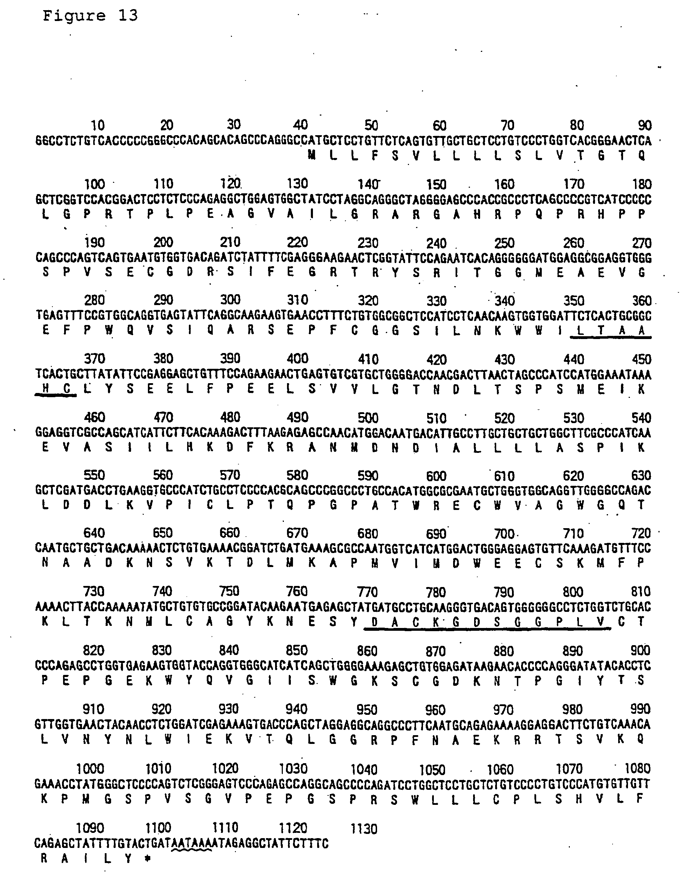

The human "Tespec PRO-3" cDNA consists of 1123 nucleotides and is predicted to encode 352 amino acids (Figure 13). This gene has a putative signal peptide at its N-terminus, and contains the "Trypsin His" and "Trypsin-Ser" motifs. In addition, cysteine residues that are predicted to form an intramolecular a disulfide bond are well conserved relative to other serine proteases.

### Example 10. Cloning of full-length cDNA of the mouse "Tespec PRO-3" gene

Mouse "Tespec PRO-3", which is the mouse counterpart of the above-mentioned human "Tespec PRO-3" is considered to contain some of the nucleotide sequences of the above-mentioned ESTs, which are derived from mouse testis. Mouse ESTs for this gene, eight sequences in total, have been deposited in a database. Among them, four ESTs are derived from the testis, one is derived from the kidney and the remaining three are derived from cDNAs of unknown origins. Thus, primers were designed on the basis of these ESTs to conduct RACE using mouse testis Marathon ready cDNA as a template, and the full-length cDNA sequence of mouse "Tespec PRO-3" was cloned.

On the basis of the nucleotide sequences of the mouse ESTs (AA497965, AA497934, AA497919, AA497949, AA271404, AA238183, AA240375, and AA105229), primers for 5' RACE, i.e. mPR03-B (SEQ ID NO: 42/ 5'-CAC CTA CTG CCA GGA TCT GTG G-3') and mPR03-D (SEQ ID NO: 43/ 5'-GGC TAT TTT CTC AAT CCA CAG GGT A-3'), and primers for 3' RACE, i.e. mPR03-A (SEQ ID NO: 44/ 5'-ATA GAG TGG GAG GAA TGC TTA CAG A-3') and mPR03-C (SEQ ID NO: 45/ 5'-GCT ACG ATG CTT GCC AGG GTG-3'), were generated. Nested RACE was conducted by using the mouse testis Marathon ready cDNA (CLONTECH) as a template, according to the manufacturer's instruction. The product obtained by RACE was sequenced directly or after subcloned into the pGEM T easy vector. The nucleotide sequence is shown in SEQ ID NO: 7 and the amino acid sequence is shown in SEQ ID NO: 8.

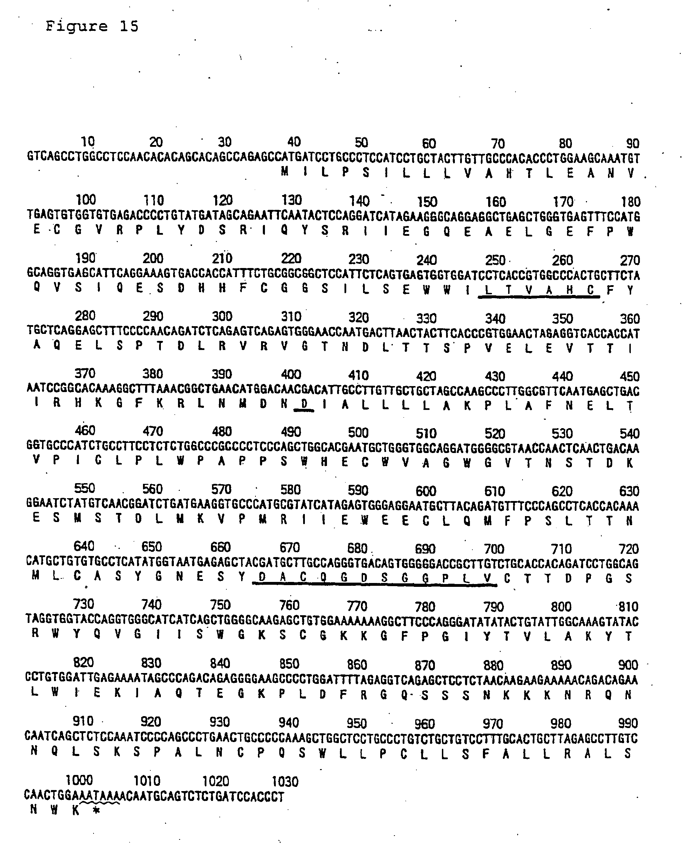

The mouse "Tespec PRO-3" cDNA consists of 1028 nucleotides and it is predicted to encode 321 amino acids (Figure 15). While the deduced amino acid sequence contains a "Trypsin-Ser" motif, it has the "Trypsin-His" motif that is deviated from the consensus motif consisting of 6 amino acids [LIVM]-[ST]-A-[STAG]-H-C at one amino acid residue (LTVAHC). However, like mouse "Tespec PRO-2", some known trypsin-family serine proteases have sequences containing several amino acid deviation in the consensus sequence, and therefore mouse "Tespec PRO-3" is predicted to function as a protease. In addition, it has a hydrophobic region predicted to be a signal peptide at its N-terminus. Cysteine residues predicted to form an intramolecular disulfide bond are well conserved in the sequence relative to other serine proteases.

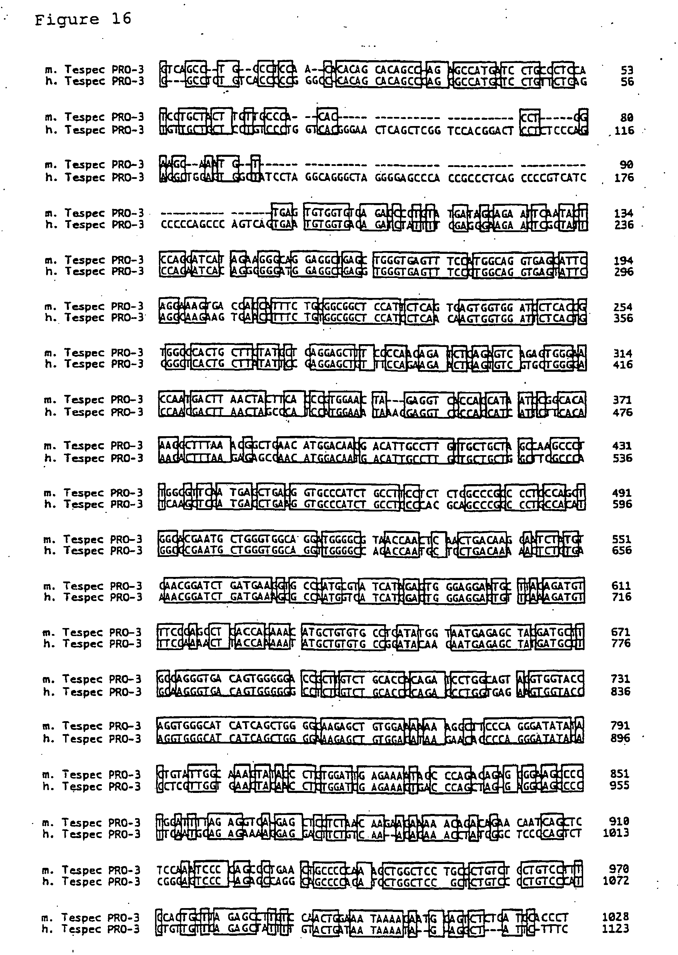

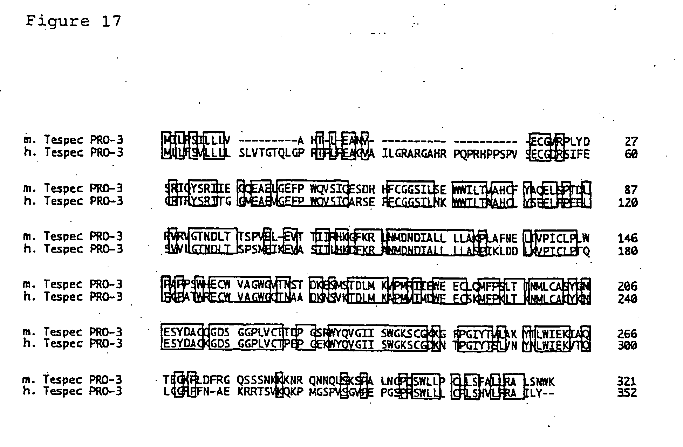

Homology between human and mouse "Tespec PRO-3" is 70.2% at the nucleotide level and 59.6% at the amino acid level (Figures 16 and 17). It was revealed that compared to human "Tespec PRO-3", mouse "Tespec PRO-3" is shorter in nucleotide sequence by about 100 residues at the 5' end, and also shorter in amino acid sequence by about 30 residues at the N-terminus.

### Industrial Applicability

Provided by the present invention are novel trypsin-family serine proteases and the genes encoding them. The proteins of the present invention were suggested to be involved in sperm differentiation and maturation or in sperm function (fertilization) . Thus, the proteases of the present invention and the genes thereof are expected to serve for developing new therapeutic or diagnostic agents for infertility and for developing new contraceptives.

## Claims

1. A DNA molecule selected from the group consisting of:
(a) a DNA comprising the nucleotide sequence shown in SEQ ID NO:7 or SEQ ID NO:9;
(b) a DNA encoding a protein comprising the amino acid sequence shown in SEQ ID NO:8 or SEQ ID NO:10;
(c) a DNA encoding a protein comprising the amino acid sequence shown in SEQ ID NO:8 or SEQ ID NO:10, wherein one or more amino acids are deleted, added, inserted and/or substituted with different amino acids and said protein having serine protease activity;
(d) a DNA encoding a partial peptide of the protein of (b) and (c) and said peptide having serine protease activity;
(e) a DNA that specifically hybridizes under stringent conditions to a DNA of any one of (a) to (d) and encoding a protein having serine protease activity.

2. A nucleotide sequence comprising at least 15 nucleotides of the nucleotide sequence of SEQ ID NO:7 or SEQ ID NO:9

3. A vector containing the DNA of claim 1.

4. A transformed host cell having the DNA according to claim 1 or the vector of claim 3 in an expressible form.

5. A method for producing a protein encoded by the DNA of claim 1 or the vector of claim 3, said method comprising the steps of: culturing the transformant according to claim 4, and recovering the expressed protein from the transformant or the culture supernatant thereof.

6. A protein encoded by the DNA of claim 1 or obtainable by the method of claim 5.

7. A fusion protein comprising the first protein according to claim 6, fused with a second peptide.

8. A DNA encoding the fusion protein of claim 7.

9. A method of screening for a substrate of the protein of claim 6, said method comprising the following steps of:
(a) contacting a test sample with said protein;
(b) detecting the protease activity of said protein against the test sample; and
(c) selecting a compound that is digested or cleaved by said protease.

10. A method of screening for a compound capable of inhibiting the activity of the protein of claim 6, said method comprising the following steps of:
(a) contacting said protein with the substrate of claim 9 in the presence of a test sample;
(b) detecting the protease activity of the protein against the substrate; and
(c) selecting a compound that reduces the protease activity relative to the protease activity detected in the absence of the test sample.

11. An antibody that binds to the protein of claim 6.

12. A method for detecting or assaying the protein according to claim 6, said method comprising the steps of contacting the antibody according to claim 11 with a test sample that is anticipated to contain the protein; and detecting or assaying formation of the immune-complex between the antibody and the protein.

## Patentansprüche

1. DNA-Molekül ausgewählt aus der Gruppe bestehend aus:
(a) einer DNA, umfassend die Nucleotidsequenz wie in SEQ ID NO: 7 oder SEQ ID NO:9 gezeigt;
(b) einer DNA, welche ein Protein codiert, das die Aminosäuresequenz wie in SEQ ID NO:8 oder SEQ ID NO:10 gezeigt umfasst;
(c) einer DNA, welche ein Protein codiert, das die Aminosäuresequenz wie in SEQ ID NO:8 oder SEQ ID NO:10 gezeigt umfasst, wobei eine oder mehre Aminosäuren deletiert, hinzugefügt, inseriert und/oder ersetzt sind mit unterschiedlichen Aminosäuren, und wobei das Protein Serinproteaseaktivität hat;
(d) einer DNA, welche ein Teilpeptid des Proteins von (b) und (c) codiert, und wobei das Peptid Serinproteaseaktivität hat;
(e) einer DNA, welche unter stringenten Bedingungen spezifisch an eine DNA von einem aus (a) bis (d) hybridisiert und ein Protein codiert, welches Serinproteaseaktivität hat.

2. Nucleotidsequenz, welche mindestens 15 Nucleotide der Nucleotidsequenz von SEQ ID NO:7 oder SEQ ID NO:9 umfasst.

3. Vektor, umfassend die DNA nach Anspruch 1.

4. Transformierte Wirtszelle, welche die DNA nach Anspruch 1 oder den Vektor nach Anspruch 3 in exprimierbarer Form trägt.

5. Verfahren zur Herstellung eines Proteins, welches von der DNA nach Anspruch 1 oder dem Vektor nach Anspruch 3 codiert wird, wobei das Verfahren die Schritte umfasst: Züchten der Transformante nach Anspruch 4 und Gewinnen des exprimierten Proteins von der Transformante oder dem Kulturüberstand davon.

6. Protein, welches von der DNA nach Anspruch 1 codiert wird oder erhältlich ist durch das Verfahren nach Anspruch 5.

7. Fusionsprotein, umfassend das erste Protein nach Anspruch 6, welches mit einem zweiten Peptid fusioniert ist.

8. DNA, welche das Fusionsprotein nach Anspruch 7 codiert.

9. Verfahren zum Screenen auf ein Substrat für das Protein nach Anspruch 6, wobei das Verfahren die folgenden Schritte umfasst:
(a) Inkontaktbringen einer Testprobe mit dem Protein;
(b) Nachweisen der Proteaseaktivität des Proteins gegen die Testprobe; und
(c) Auswählen einer Verbindung, welche durch die Protease verdaut oder geschnitten wird.

10. Verfahren zum Screenen auf eine Verbindung, welche in der Lage ist, die Aktivität des Proteins nach Anspruch 6 zu hemmen, wobei das Verfahren die folgenden Schritte umfasst:
(a) Inkontaktbringen des Proteins mit dem Substrat nach Anspruch 9 in Gegenwart einer Testprobe;
(b) Nachweisen der Proteaseaktivität des Proteins gegen das Substrat; und
(c) Auswählen einer Verbindung, welche die Proteaseaktivität reduziert gegenüber der Proteaseaktivität, welche in Abwesenheit der Testprobe nachgewiesen wird.

11. Antikörper, welcher an das Protein nach Anspruch 6 bindet.

12. Verfahren zum Nachweisen oder Testen des Proteins nach Anspruch 6, wobei das Verfahren die Schritte umfasst: Inkontaktbringen des Antikörpers nach Anspruch 11 mit einer Testprobe, von der angenommen wird, dass sie das Protein enthält; und Nachweisen oder Testen der Bildung des Immunkomplexes zwischen dem Antikörper und dem Protein.

## Revendications

1. Molécule d'ADN choisie dans le groupe consistant en :
(a) un ADN comprenant la séquence nucléotidique illustrée par SEQ ID NO : 7 ou SEQ ID NO : 9 ;
(b) un ADN codant pour une protéine comprenant la séquence d'acides aminés illustrée par SEQ ID NO : 8 ou SEQ ID NO : 10 ;
(c) un ADN codant pour une protéine comprenant la séquence d'acides aminés illustrée par SEQ ID NO : 8 ou SEQ ID NO:10, dans laquelle un ou plusieurs acides aminés sont délétés, ajoutés, insérés et/ou substitués par des acides aminés différents et ladite protéine possédant une activité sérine protéase ;
(d) un ADN codant pour un peptide partiel de la protéine de (b) et (c) et ledit peptide possédant une activité sérine protéase ;
(e) un ADN qui s'hybride spécifiquement dans des conditions stringentes à un ADN de l'un quelconque de (a) à (d) et codant pour une protéine possédant une activité sérine protéase.

2. Séquence nucléotidique comprenant au moins 15 nucléotides de la séquence nucléotidique de SEQ ID NO : 7 ou SEQ ID NO : 9.

3. Vecteur contenant l'ADN selon la revendication 1.

4. Cellule hôte transformée ayant l'ADN selon la revendication 1 ou le vecteur selon la revendication 3 sous une forme exprimable.

5. Procédé de production d'une protéine codée par l'ADN selon la revendication 1 ou le vecteur selon la revendication 3, ledit procédé comprenant les étapes consistant à : cultiver le transformant selon la revendication 4 et récupérer la protéine exprimée à partir du transformant ou du surnageant de la culture de celui-ci.

6. Protéine codée par l'ADN selon la revendication 1 ou pouvant être obtenue par le procédé selon la revendication 5.

7. Protéine de fusion comprenant la première protéine selon la revendication 6, fusionnée à un second peptide.

8. ADN codant pour la protéine de fusion selon la revendication 7.

9. Procédé de criblage d'un substrat de la protéine selon la revendication 6, ledit procédé comprenant les étapes suivantes consistant à :
(a) mettre en contact un échantillon à tester avec ladite protéine ;
(b) détecter l'activité protéase de ladite protéine contre l'échantillon à tester ; et
(c) sélectionner un composé qui est digéré ou clivé par ladite protéase.

10. Procédé de criblage d'un composé capable d'inhiber l'activité de la protéine selon la revendication 6, ledit procédé comprenant les étapes suivantes consistant à :
(a) mettre en contact ladite protéine avec le substrat selon la revendication 9 en présence d'un échantillon à tester ;
(b) détecter l'activité protéase de la protéine contre le substrat ; et
(c) sélectionner un composé qui réduit l'activité protéase par rapport à l'activité protéase détectée en l'absence de l'échantillon à tester.

11. Anticorps qui se lie à la protéine selon la revendication 6.

12. Procédé de détection ou d'analyse de la protéine selon la revendication 6, ledit procédé comprenant les étapes consistant à mettre en contact l'anticorps selon la revendication 11 avec un échantillon à tester qui est supposé contenir la protéine ; et détecter ou analyser la formation du complexe immun entre l'anticorps et la protéine.
